(19) Europäisches Patentamt
European Patent Office
Office européen des brevets

(11) EP 0 559 690 B1

(12) **EUROPÄISCHE PATENTSCHRIFT**

(45) Veröffentlichungstag und Bekanntmachung des Hinweises auf die Patenterteilung:
**04.06.1997 Patentblatt 1997/23**

(21) Anmeldenummer: **91920328.1**

(22) Anmeldetag: **27.11.1991**

(51) Int Cl.6: **C07J 53/00**, C07J 63/00, A61K 31/56

(86) Internationale Anmeldenummer:
**PCT/EP91/02239**

(87) Internationale Veröffentlichungsnummer:
**WO 92/09618 (11.06.1992 Gazette 1992/13)**

(54) **8-EN-19, 11 beta-ÜBERBRÜCKTE STEROIDE, DEREN HERSTELLUNG UND DIESE ENTHALTENDE PHARMAZEUTISCHE PRÄPARATE**

8-ENE-19, 11 beta-BRIDGED STEROIDS, THEIR PREPARATION AND PHARMACEUTICAL FORMULATIONS CONTAINING THEM

STEROIDES 8-ENE-19, 11 beta PONTES, LEUR PRODUCTION ET COMPOSITIONS PHARMACEUTIQUES LES CONTENANT

(84) Benannte Vertragsstaaten:
**AT BE CH DE DK ES FR GB GR IT LI LU NL SE**

(30) Priorität: **27.11.1990 DE 4038128**

(43) Veröffentlichungstag der Anmeldung:
**15.09.1993 Patentblatt 1993/37**

(73) Patentinhaber: **SCHERING AKTIENGESELLSCHAFT**
**13342 Berlin (DE)**

(72) Erfinder:
- **OTTOW, Eckhardt**
  **D-1000 Berlin 45 (DE)**
- **NEEF, Günter**
  **D-1000 Berlin 31 (DE)**
- **ELGER, Walter**
  **D-1000 Berlin 33 (DE)**
- **SCHNEIDER, Martin**
  **D-1000 Berlin 28 (DE)**
- **FRITZEMEIER, Karl-Heinrich**
  **D-1000 Berlin 27 (DE)**

(56) Entgegenhaltungen:
**EP-A- 0 283 428** **EP-A- 0 360 369**
**DE-A- 3 717 169**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 559 690 B1

**Beschreibung**

Die vorliegende Erfindung betrifft den in den Patentansprüchen gekennzeichneten Gegenstand, das heißt neue 8-En-19,11β-überbrückte Steroide, Verfahren zu ihrer Herstellung, diese Verbindungen enthaltende pharmazeutische Präparate, ihre Verwendung zur Herstellung von Arzneimitteln sowie die dazu benötigten neuen Zwischenprodukte.

Die erfindungsgemäßen 8-En-19,11β-überbrückten Steroide werden durch die allgemeine Formel I beschrieben

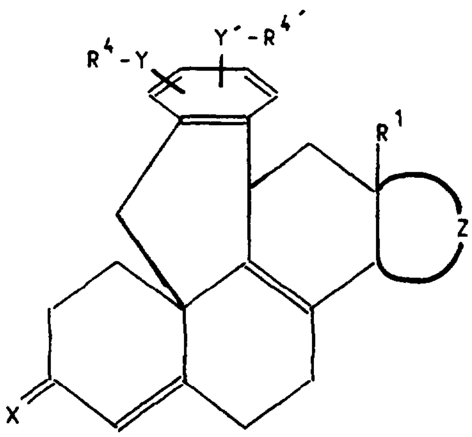

(I),

worin

$R^1$ für einen Methyl- oder Ethylrest,
X für ein Sauerstoffatom, eine Hydroxyiminogruppe oder zwei Wasserstoffatome,
Z für den Rest eines Ringes der Formel

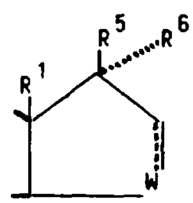

steht, worin
$R^1$ die in Formel I genannte Bedeutung hat, die von W ausgehende gestrichelte Linie die etwaige Anwesenheit einer Doppelbindung bedeutet,
W einen $CH_2$-, CH-, $CH_2CH_2$- oder $CHCH_2$-Rest,
$R^5/R^6$ $-OR^7/-C{\equiv}C-U$

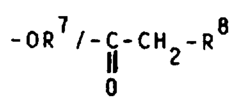

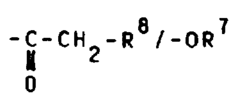

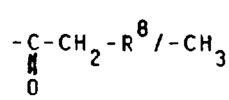

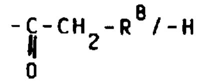

$-OR^7/-(CH_2)_m-CH_2-R^9$

$-OR^7/-CH{=}CH(CH_2)_k-CH_2-R^9$

$-OR^{10}/-(CH_2)_k-C{\equiv}C-U$

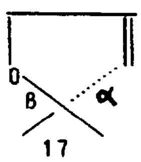

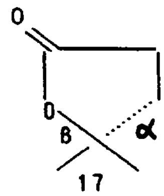

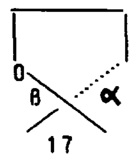

mit $R^7$ in der Bedeutung eines Wasserstoffatoms, eines Alkyl- oder Acylrestes mit 1 bis 4 Kohlenstoffatomen, U in der Bedeutung eines Wasserstoffatoms, einer Alkyl, Hydroxyalkyl-, Alkoxyalkyl-, Acyloxyalkylgruppe mit jeweils 1 bis 4 Kohlenstoffatomen im Alkyl- bzw. Acylrest oder eines Halogenatoms,
$R^8$ in der Bedeutung eines Wasserstoffatoms, einer Hydroxygruppe, einer Alkyl-, O-Alkyl- oder O-Acylgruppe mit jeweils 1 bis 4 Kohlenstoffatomen,
$R^9$ in der Bedeutung eines Wasserstoffatoms, eines Hydroxy- oder Cyanidrestes, einer O-Alkyl- oder O-Acylgruppe mit jeweils 1 bis 4 Kohlenstoffatomen,
$R^{10}$ in der Bedeutung eines Wasserstoffatoms, einer Alkyl- oder Acylgruppe mit jeweils 1 bis 10 Kohlenstoffatomen,
m in der Bedeutung 0, 1, 2 oder 3,
k in der Bedeutung 0, 1 oder 2,

bedeuten,
worin

$R^4$ und $R^4$, die gleich oder verschieden sind, jeweils für ein Wasserstoffatom, einen Cyanidrest, eine $-OR^{11}$-, -S $(O)_kR^{11}$-, $-N(O)_nR^{11}R^{12}$-, $-O-SO_2R^{13}$-, $-P(O)(OR^{14})_2$, $-SiR^{14}_3$ oder $-SnR^{14}_3$-Gruppe steht mit k in der Bedeutung der Ziffern 0, 1 oder 2, n in der Bedeutung der Ziffern 0 oder 1,
$R^{11}$ in der Bedeutung eines Wasserstoffatoms oder eines $C_1$-$C_8$-Alkylrestes,
$R^{12}$ in der Bedeutung von $R^{11}$, eines Cyanid- oder eines $C_1$-$C_{10}$-Acylrestes,
$R^{13}$ in der Bedeutung eines perfluorierten $C_1$-$C_4$-Alkylrestes,
$R^{14}$ in der Bedeutung eines $C_1$-$C_4$ -Alkylrestes oder
$R^{11}$ und $R^{12}$ innerhalb einer $-N(O)_n$ $R^{11}R^{12}$-Gruppe gemeinsam unter Einschluß von N einen 5- oder 6-gliedrigen heterocyclischen Ring bilden, wobei im Ring noch ein weiteres Heteroatom N, O oder S enthalten sein kann, Y und Y', die gleich oder verschieden sind, jeweils eine direkte Bindung, eine geradkettige oder verzweigte, gegebenenfalls Doppel- oder Dreifachbindung(en) aufweisende Alkylengruppe mit bis zu 20 Kohlenstoffatomen, die gegebenenfalls substituiert ist durch eine oder mehrere Oxo-, $C_1$-$C_{10}$-Acyloxy--, $-OR^{11}$-, $-S(O)_kR^{11}$- und/oder -N $(O)_nR^{11}R^{12}$-Gruppe(n), einen gegebenenfalls substituierten carbocylischen oder heterocyclischen Arylrest oder $R^4$-Y und $R^{4'}$-Y' gemeinsam den Rest eines, gegebenenfalls substituierten gesättigten, ungesättigten oder aromatischen 5-oder 6-gliedrigen Ringes mit 0 bis 2 Sauerstoff-, Schwefelatomen und/oder $NR^{11}$-Gruppen bedeuten, mit der Maßgabe, daß nur dann k und n größer als 0 sind, wenn $R^{11}$ für einen $C_1$-$C_8$-Alkylrest steht,

sowie gegebenenfalls deren pharmazeutisch verträgliche Additionssalze mit Säuren.

Bei der Substitution des Phenylringes ist die Monosubstitution in 3-, 4-oder 5-Position sowie die Disubstitution in 4- und 5- oder 3- und 4-Position unter Bildung eines ankondensierten zweiten Ringes, zum Beispiel eines Cyclohexen-, Pyrrol-, Furyl-, Pyrrolin-, 1,3-Dioxacyclopenten-, Pyrazolin-, Didehydromorpholin-, Didehydropiperidin-, Didehydropiperazin-, Dihydropyran-, Pyrimidin-, Pyridin-, Pyrazin-, 1,4-Dioxacyclohexen-Ringes, bevorzugt.

Die für $R^1$ und $R^{11}$ bzw. $R^2$, $R^3$, B, G und D stehenden Alkylreste sollen im Fall von $R^1$ 1 oder 2, im Fall von $R^{11}$

1 bis 8 und ansonsten 1 bis 4 Kohlenstoffatome tragen, wobei die Methyl-, Ethyl-, Propyl-, Isopropyl-, Butyl-bzw. Methyl-, Ethyl-, Propylgruppen bevorzugt sind.

Steht $R^{12}$ für einen Acylrest, so ist die Formyl-, Acetyl-, Propionyl-, Butyryl und Benzoyl-gruppe bevorzugt.

$R^{11}$ und $R^{12}$ stehen auch gemeinsam unter Einschluß des Stickstoffatoms für einen heterocyclischen Fünf- oder Sechsring, der außer N- und C-Atome auch noch zusätzlich ein 0- oder S-Atom enthalten kann; beispielsweise genannt seien der Pyrrol-, Pyrrolidin-, Piperidin-, Piperazin-, Morpholin-, Oxa-und Thiazolidin- sowie Thiadiazolidin-Ring.

Die in $R^5$ und $R^6$ bzw. $R^7$, $R^8$, $R^9$, $R^{10}$ und U der allgemeinen Formel I enthaltenen Alkyl-, Alkoxy- sowie Acyloxygruppen sollen jeweils 1 bis 4 Kohlenstoffatome enthalten, wobei die Methyl-, Ethyl-, Propyl-, Isopropyl-, Methoxy-, Ethoxy-Propoxy-, Isopropoxy-, Formyl-, Acetyl-, Propionyl- und Isopropionylgruppe bevorzugt sind.

Von den Alkenylresten in $R^6$ sind die Propenyl- und Butenylgruppe, die in der E-oder Z-Konfiguration vorliegen können, bevorzugt, d.h. wenn $R^6$ für $-CH{=}CH(CH_2)_kCH_2-R^9$ steht, dann soll k bevorzugt 0 oder 1 bedeuten.

Als gegebenenfalls substituierter carbocyclischer oder heterocyclischer Arylrest kommen ein Phenyl-, Naphthyl-, 2-Methoxyphenyl-, 3-Methoxyphenyl-, 4-Methoxyphenyl-, 2-Tolyl-, 3-Tolyl-, 4-Tolyl-, 2-Dimethylaminophenyl-, 3-Dimethylaminophenyl-, 4-Dimethylaminophenyl-, Furyl-2-, Furyl-3-, Thienyl-2-, Thienyl-3-, Pyridyl-2-, Pyridyl3-, Pyridyl-4-, Pyrimidinyl, Thiazolyl-, Imidazolyl-Rest infrage.

Folgende Verbindungen sind erfindungsgemäß bevorzugt:

17β-Hydroxy-11β,19-(4-methoxy-o-phenylen)-17α-(prop-1-inyl)-4,8-androstadien-3-on
11β,19-(Ethyl-o-phenylen)-17β-hydroxy-17α-(prop-1-inyl)-4,8-androstadien3-on
11β,19-[4-(4-Cyanphenyl)-o-phenylen]-17β-hydroxy-17α-(prop-1-inyl)-4,8-androstadien-3-on
11β,19-[4-(4-Cyanphenyl)-o-phenylen]-17α-(ethinyl)-17β-hydroxy-4,8-androstadien-3-on
11β,19-[4-(4-Cyanphenyl)-o-phenylen]-17β-hydroxy-17α-(3-hydroxyprop-1-inyl)-4,8-androstadien-3-on
11β,19-[4-(4-Cyanphenyl)-o-phenylen]-17β-hydroxy-17α-(3-hydroxyprop-1(Z)-enyl)-4,8-androstadien-3-on
17β-Hydroxy-17α-(prop-1-inyl)-11β,19-[4-(5-pyrimidinyl)-o-phenylen]-4,8-androstadien-3-on
17β-Hydroxy-17α-(3-hydroxyprop-1-inyl)-11β,19-[4-(3-pyridyl)-o-phenylen]-4,8-androstadien-3-on
17β-Hydroxy-17α-(3-hydroxyprop-1-inyl)-11β,19-[4-(4-methoxyphenyl)-o-phenylen]-4,8-androstadien-3-on
17β-Hydroxy-17α-(3-hydroxyprop-1-inyl)-11β,19-[4-(4-methylthiophenyl)-o-phenylen]-4,8-androstadien-3-on
11β,19-(4-Acetyl-o-phenylen)-17β-hydroxy-17α-(3-hydroxyprop-1-inyl)-4,8-androstadien-3-on
17β-Hydroxy-17α-(3-hydroxyprop-1(Z)-enyl)-11β,19-[4-(3-pyridyl)-o-phenylen]-4,8-androstadien-3-on
17β-Hydroxy-17α-(3-hydroxyprop-1(Z)-enyl)-11β,19-[4-(4-methoxyphenyl)-o-phenylen]-4,8-androsstadien-3-on
17β-Hydroxy-17α-(3-hydroxyprop-(Z)-enyl)-11β,19-[4-(4-methylthiophenyl)-o-phenylen]-4,8-androstadien-3-on
11β,19-(4-Acetyl-o-phenylen)-17β-hydroxy-17α-(3-hydroxyprop-1(Z)-enyl)-4,8-androstadien-3-on
11β,19-(4-Acetyl-o-phenylen)-17β-hydroxy-17α-(prop-1-inyl)-4,8-androstadien-3on
17β-Hydroxy-11β,19-[4-(4-methylthiophenyl)-o-phenylen]-17α-(prop-1-inyl)-4,8-androstadien-3-on
17β-Hydroxy-11β,19-[4-(3-furanyl)-o-phenylen]-17α-(prop-1-inyl)-4,8-androstadien-3-on
17β-Hydroxy-17α-(prop-1-inyl)-11β,19-[4-(3-pyridyl)-o-phenylen]-4,8-androstadien-3-on
17β-Hydroxy-17α-methyl-11β,19-[4-(4-cyanphenyl)-o-phenylen]-4,8-androstadien-3-on
17α-Cyanmethyl-17β-hydroxy-11β,19-[4-(4-cyanphenyl)-o-phenylen]-4,8-androstadien-3-on
17α-Cyanmethyl-17β-hydroxy-11β,19-[4-(4-dimethylaminophenyl)-o-phenylen]-4,8-androstadien-3-on
17β-Hydroxy-17α-(3-hydroxyprop-1(Z)-enyl)11β,19-(4-vinylphenyl-o-phenylen)-4,8-androstadien-3-on
17β-Hydroxy-11β,19-(4-methylthiophenyl-o-phenylen)-17α-(prop-1-inyl)-4,8-androstadien-3-on
17β-Hydroxy-17α-(3-hydroxyprop-1(Z)-enyl)-11β,19-(4-methylsulfinylphenyl-o-phenylen)-4,8-androstadien-3-on
17β-Hydroxy-17α-(3-hydroxyprop-1(Z)-enyl)-11β,19-(4-methylthio-o-phenylen)-4,8-androstadien-3-on
17β-Hydroxy-17α-(3-hydroxyprop-1(Z)-enyl)-11β,19-(4-methylsulfinyl-o-phenylen)-4,8-androstadien-3-on
11β,19-(4-Dimethylamino-o-phenylen)-17β-hydroxy-17α-(3-hydroxyprop-1(Z)-enyl)-4,8-androstadien-3-on
11β,19-[4-(4-Dimethylaminophenyl)-o-phenylen]-17β-hydroxy-17α-(3-hydroxyprop-1(Z)-enyl)-4,8-androstadien-3-on
11β,19-[4-(3-Furyl)-o-phenylen]-17β-hydroxy-17α-(3-hydroxyprop-1(Z)-enyl)-4,8-androstadien-3-on
11β,19-[4-(4-Cyanphenyl)-o-phenylen]spiro[androsta-4,8-dien-17β,2'(5'H)-furan]-3-on
11β,19-[4-(4-Cyanphenyl)-o-phenylen]-4',5'-dihydrospiro[androsta-4,8-dien-17β,2'(3'H)-furan]-3-on
11β,19-[4-(4-Acetylphenyl)-o-phenylen]-17β-hydroxy-17α-(3-hydroxyprop-1-inyl)-4,8-androstadien-3-on
11β,19-[4-(4-Acetylphenyl)-o-phenylen]-17β-hydroxy-17α-(3-hydroxyprop-1(Z)enyl)-4,8-androstadien-3-on
11β,19-[4-(4-Acetylphenyl)-o-phenylen]spiro[androsta-4,8-dien-17β,2'(5'H)-furan]-3-on
11β,19-[4-(4-Acetylphenyl)-o-phenylen]-4',5'-dihydrospiro[androsta-4,8-dien-17β,2'-(3'H)-furan]-3-on
11β,19-[4-(4-Methylthiophenyl)-o-phenylen]spiro[androsta-4,8-dien-17β,2'(5'H)-furan]-3-on
11β,19-[4-(4-Methylthiophenyl)-o-phenylen]-4',5'-dihydrospiro[androsta-4,8-dien-17β,2'-(3'H)-finan]-3-on
11β,19-(4-Acetyl-o-phenylen)spiro[androsta-4,8-dien-17β,2'(5'H)-furan]-3-on
11β,19-(4-Acetyl-o-phenylen)-4',5'-dihydrospiro[androsta-4,8-dien-17β,2'(3'H)-furan]-3-on.

11β-Phenyl-14βH-steroide, die wie die erfindungsgemäßen Verbindungen der allgemeinen Formel I eine Methylenbrücke zwischen dem 10C-Atom des Steroidgerüstes und einem ortho-C-Atom des Phenylringes (ortho-ständig zur Verknüpfung zum Steroid) aufweisen können, sind bereits in der EP-A 0 360 369 beschrieben. Abweichend von den vorliegenden Verbindungen weisen sie lediglich eine 8,9-Einfachbindung und die unnatürliche 14β-Konformation auf.

Die neuen Verbindungen der allgemeinen Formel I werden erfindungsgemäß nach dem Verfahren gemäß Anspruch 13 hergestellt.
Dabei wird eine Verbindung der allgemeinen Formel II

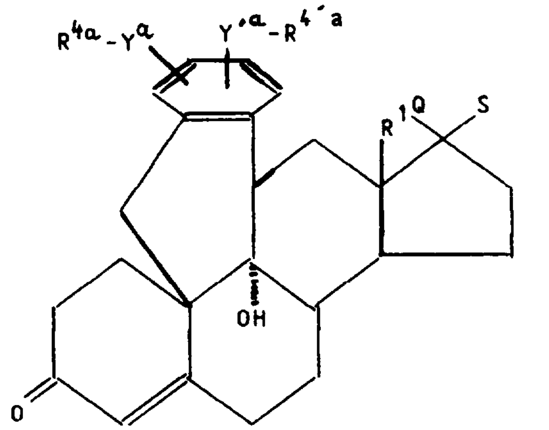

(II),

worin

$R^{4a}$, $R^{4'a}$, $Y^a$ und $Y'^a$ a die gleiche Bedeutung wie $R^4$, $R^{4'}$, Y und Y' haben, wobei gegebenenfalls vorhandene Hydroxy-, Mercapto-, Amino-, Oxo- und/ oder terminale Acetylengruppen geschützt sind, oder $R^{4a}$-$Y^a$- und/oder $R^{4'a}$-$Y'^a$ je eine Methoxy-, Hydroxy- oder Perfluoralkylsulfonatgruppe,
Q und S gemeinsam ein Sauerstoffatom oder eine der unter $R^5/R^6$ genannten Substituentenkombinationen die den nachfolgenden Reaktionsschritt der Wasserabspaltung unbeschadet übersteht oder in welchem freie Hydroxygruppen geschützt sind,

bedeuten,
durch Umsetzung mit einem wasserentziehenden Mittel in die entsprechende 8-En-Verbindung der allgemeinen Formel III

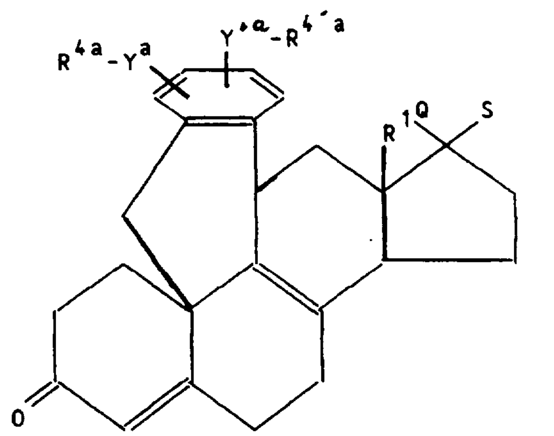

(III),

überführt wird
und anschließend gegebenenfalls

wenn Q und S gemeinsam ein Sauerstoffatom bedeuten, die 3-Ketofunktion selektiv in Form eines entsprechenden Dienolethers, Ketals oder Dithioketals geschützt und die Substituenten $R^5/R^6$ oder deren Vorläufer durch nucleophile Addition an die 17-Ketofunktion in an sich bekannter Weise (Seitenkettenaufbau) eingeführt, die 3-Ketogruppe wieder freigesetzt, danach gegebenenfalls, wenn $R^6$ $-(CH_2)_m-CH_2-R^9$ oder $-CH=CH-(CH_2)_kCH_2-R^9$ bedeuten soll, die entsprechende Alkinylverbindung katalytisch hydriert sowie gegebenenfalls
wenn $R^{4a}-Y^a-$ und/oder $R^{4'a}-Y'^a-$ eine Perfluoralkylsulfonatgruppe bedeutet, die gegebenenfalls zuvor aus einer Methoxy- über eine Hydroxygruppe generiert wurde, die Perfluoralkylsulfonatverbindung entweder direkt oder durch Austausch der Perfluoralkylsulfonatabgangsgruppe gegen eine Zinntrialkylgruppe über die entsprechende Zinntrialkylverbindung in eine Verbindung überführt, die im 19,11β-Phenylring, gegebenenfalls nach weiteren Reaktionen, das gewünschte Substitutionsmuster aufweist, wobei dieser zuletzt genannte Verfahrensschritt der Arylkupplung prinzipiell auf jeder beliebigen Stufe des erfindungsgemäßen Verfahrens durchgeführt werden kann, das so erhaltene Produkt gegebenenfalls von Schutzgruppen befreit, gewünschtenfalls die im 19,11β-Phenylenring gegebenenfalls enthaltene(n) Hydroxy-, Mercapto und/oder Aminogruppe(n) alkyliert bzw. acyliert, gewünschtenfalls die in dem(n) Arylsubstituenten gegebenenfalls enthaltene(n) Amino- und/oder Sulfidgruppe(n) oxidiert, gewünschtenfalls mit Hydroxylamin-hydrochlorid zum Produkt der allgemeinen Formel I mit X in der Bedeutung der Hydroxyiminogruppierung $>N\sim OH$ umgesetzt sowie gegebenenfalls ein pharmazeutisch verträgliches Säureadditionssalz hergestellt.

Vorzugsweise wird Thionylchlorid als wasserentziehendes Mittel zur Abspaltung der 9α-Hydroxygruppe verwendet.

Die vorstehend genannten Hydroxy-, Mercapto- und Keto-Schutzgruppen sind im sauren Milieu leicht abspaltbare Gruppen, wie zum Beispiel die Methoxymethyl-, Ethoxymethyl-,Tetrahydropyranyl-, Ethylendioxyketal-, Ethylendithioketal-oder 2,2-Dimethyltrimethylendioxyketalgruppe. Eine (oder mehrere) am 19-Phenylring vorhandene Hydroxygruppe(n) wird (werden) durch eine basisch zu entfernende Gruppe, beispielsweise durch eine Methoxygruppe, geschützt. Diese kann beispielsweise durch Reaktion mit Natriumthiophenolat wieder abgespalten werden.
Schutzgruppen für Amino- und terminale Acetylengruppen (zum Beispiel die Tri-methylsilyl- und tert.-Butyldimethylsilylgruppe) sind dem Fachmann ebenfalls bekannt und werden nach der gewünschten Reaktionsfolge auch nach literaturbekannten Verfahren gespalten (Synthesis 1980, 627, J. Org. Chem. 46 (1986) 2280).

Der 17-Seitenkettenaufbau für die letzlich gewünschten Endprodukte der allgemeinen Formel I erfolgt analog literaturbekannten Verfahren (zum Beispiel J. Fried, J.A. Edwards, "Organic Reactions in Steroid Chemistry", Van Nostrand Reinhold Company 1972, Vol. 1 und 2; "Terpenoids and Steroids", Specialist Periodical Report, The Chemical Society, London, Vol. 1-12)

Die für die Herstellung fast aller Endprodukte nötige Oxidation der 17β-Hydroxygruppe wird in an sich bekannter Weise, zum Beispiel durch Oppenauer-Oxidation oder mit Chromsäurereagenzien (Jones'-Reagenz oder Chromsäure-Pyridin) durchgeführt.

Die Freisetzung der 3-Ketofunktion unter gleichzeitiger Wasserabspaltung und Ausbildung der 4(5)-Doppelbindung erfolgt durch Behandlung mit Säure oder einem sauren Ionenaustauscher. Die saure Behandlung erfolgt in an sich bekannter Weise, indem man das entsprechende 5α-Hydroxy-3-ketal in einem mit Wasser mischbaren Lösungsmittel, wie wäßrigem Methanol, Ethanol oder Aceton, löst und auf die Lösung katalytische Mengen Mineral- oder Sulfonsäure, wie Salzsäure, Schwefelsäure, Phosphorsäure, Perchlorsäure oder p-Toluolsulfonsäure, oder eine organische Säure, wie Essigsäure, so lange einwirken läßt, bis vorhandene Schutzgruppen entfernt sind und gegebenenfalls Wasser abgespalten ist. Die Umsetzung, die bei Temperaturen von 0 bis 100°C abläuft, kann auch mit einem sauren Ionenaustauscher vorgenommen werden. Der Verlauf der Umsetzung kann mit analytischen Methoden, beispielsweise durch Dünnschichtchromatographie entnommener Proben, verfolgt werden.

Ein ganz besonderer Vorteil der vorliegenden Erfindung liegt in der großen Bandbreite der am 19-11β-Phenylenrest einführbaren Substituenten (M. Pereyre, J.-P. Ouintard, A. Rahm, Tin in Organic Synthesis; Butterworths, 1987). Zum einen können die im späteren Endprodukt vorhandenen Substituenten $R^4$-Y-bzw. $R^{4'}$ -Y'- direkt eingeführt werden, (vergl. EP-A 0283 428).

Mit einer weiteren Ausführungsform des erfindungsgemäßen Verfahrens gelingt es, den (die) Substituenten im 19,11β-Phenylenrest über einen weiten Bereich zu variieren, indem der (die) Substituent(en) erst unmittelbar vor oder sogar erst nach Ausbildung der 8,9-Doppelbindung eingeführt wird(werden). Dazu wird mindestens eine der im 19,11β-Phenylenrest des Ausgangsproduktes vorhandenen und geschützten Hydroxygruppe von ihrer Schutzgruppe befreit und aus der freien OH-Verbindung durch Umsetzung mit Perfluoralkylsulfonsäureanhydrid (Alkyl = $C_1$-$C_4$) nach an sich bekannten Methoden [P.J. Stang, M. Hanack and L.R. Subramanian, Synthesis 85 (1982)] die entsprechende Perfluoralkylsulfonatverbindung hergestellt.
Dabei wird entweder so vorgegangen, daß in einer übergangsmetallkatalysierten Reaktion (vorzugsweise Pd°) die Perfluorsulfonatabgangsgruppe unter im wesentlichen fast gleichzeitiger Substitution durch den gewünschten Substituenten oder dessen Vorstufe verdrängt wird (J.E. McMurry and S. Mohanraj, Tetrahedron Letters, 24, No. 27, S. 2723-2726, 1983; X. Lu und J. Zhu, Communications, S. 726-727, 1987; Q. -Y. Chen und Z.-Y. Yang, Tetrahedron Letters 27, No. 10, S. 1171-1174, 1986; S. Cacchi, P.G. Ciattini, E. Morera und G. Ortar, Tetrahedron Letters, 27, No.

33, S. 3931-3934, 1986; A.M. Echavarren und J.K. Stille, J. Am. Chem. Soc. 1987, 109, S. 5478-5486) oder es wird aus der Perfluoralkylsulfonat-Verbindung intermediär und übergangsmetallkatalysiert eine entsprechende Tri-organylstannyl-, vorzugsweise Tri--n-alkylstannyl-Verbindung hergestellt [J.K. Stille, Angew. Chem. 98 (1986), S. 504-519]. Diese wird anschließend mit einem halogen-, vorzugsweise brom- oder jodsubstituierten carbocyclischen (vergl. Vorschrift 6c) unter Ausgangsverbindungen) oder heterocyclischen Aromaten (vergl. Vorschrift 3b) Ausgangsverbindungen) [Y. Yamamoto, Y. Azuma, H. Mitoh, Communications, S. 564-565, 1986; T. J. Bailey, Tetrahedron Letters, 27, No. 37, S. 44074410, 1986], der gegebenenfalls noch weitere Substituenten tragen kann, umgesetzt; der 19,11β-Phenylenrest weist darin dann die gewünschte bzw. einen Vorläufer der gewünschten Substitution auf. Die Vorläufer werden nach gängigen Methoden der organischen Chemie zu den letztendlich gewünschten Verbindungen weiterverarbeitet. Eine aktuelle Übersicht über Arylkupplungsreaktionen findet sich in J. Organometallic Chem. 392 (1990), S. 285. Weist der 19-Phenylring zwei geschützte Hydroxygruppen auf, so kann zunächst lediglich die Schutzgruppe der einen (ersten) Hydroxygruppe selektiv entfernt werden, die freie Hydroxygruppe funktionalisiert werden, anschließend gegebenenfalls die Schutzgruppe der zweiten Hydroxygruppe abgespalten und diese Hydroxygruppe modifiziert werden, gegebenenfalls auch durch Reaktion mit der sich an der ersten Hydroxygruppe nunmehr befindenden Funktion.

Die Entfernung der 3-Oxogruppe zu einem Endprodukt der allgemeinen Formel I mit X in der Bedeutung von zwei Wasserstoffatomen kann z.B. nach der in DE-A 2805490 angegebenen Vorschrift durch Thioketalisierung und anschließende reduktive Spaltung erfolgen.

Edukte mit einem D-Homo-Steroidgerüst sind auch z.B. durch Tiffeneau-Umlagerung analog der in Australian J. Chem. 8 11955), 519 und in "Organic Reactions in Steroid Chemistry" Vol 2, 388 veröffentlichten Vorschrift zu erhalten. Die notwendigen 17α-Aminomethyl-17β-hydroxyverbindungen sind zum Beispiel über die Öffnung der 17,20-Spiroepoxide mit Ammoniak oder auch durch Lithiumaluminiumreduktion der acetylierten 17β-Hydroxy-17-cyanoverbindungen zugänglich. Die Spiroepoxide sind durch Umsatz der entsprechenden 17-Ketone mit Dimethylsulfoniummethylid in Dimethylformamid [Journal f. prakt. Chemie 314(1972), 667-668) zugänglich. Die acetylierten Cyanhydrine sind durch Anlagerung von Cyanwasserstoff an die entsprechenden 17-Ketone und anschließende Acetylierung nach bekannten Vorschriften (z.B. Australian J. Chem. 8 (1955),519) zugänglich.

Edukte mit einem ungesättigten 0-Ring sind zum Beispiel durch modifizierte Saegusa-Oxidation (Tetrahedron 42 (1986) 2971) der entsprechenden Enolverbindungen des 17-Ketons zugänglich. Zum Beispiel ist der Trimethylsilylenolether durch Überführung des 17-Ketons mit Lithiumdiisopropylamid in Tetrahydrofuran in das korrespondierende Enolat und Abfang durch Trimethylchlorsilan darstellbar (Synthesis 1983, 1). Die Einführung der Substituenten $R^5$ und $R^6$ erfolgt nach den üblichen Verfahren des C-17-Seitenkettenaufbaus durch nucleophile Addition an das - durch z.B. Oppenauer-Oxidation der C-17-Hydroxygruppe erhaltene -17-Keton und Folgereaktionen ("Terpenoids and Steroids", Specialist Periodical Report, The Chemical Society, London, Vol. 1-12)

Die Einführung des Substituenten -C≡C-U als $R^6$, wobei U die oben angegebene Bedeutung hat, erfolgt mit Hilfe einer Verbindung der allgemeinen Formel MC≡C-U, in der U eine Alkin-Schutzgruppe, wie zum Beispiel der Trimethylsilyl- oder tert.-Butyldimethylsilylrest, oder aber im Fall, wenn U eine Alkylgruppe mit 1-4 C-Atomen ist, U selbst der Rest U ist.

Die metallorganische Verbindung kann auch in situ gebildet und mit dem 17-Keton zur Reaktion gebracht werden. So kann man zum Beispiel auf das 17-Keton in einem geeigneten Lösungsmittel Acetylen und ein Alkalimetall, insbesondere Kalium, Natrium oder Lithium, in Gegenwart eines Alkohols oder in Gegenwart von Ammoniak einwirken lassen. Das Alkalimetall kann auch in Form von zum Beispiel Methyl- oder Butyllithium zur Einwirkung kommen. Als Lösungsmittel sind insbesondere Dialkylether, Tetrahydrofuran, Dioxan, Benzol und Toluol geeignet.

Die Einführung von 3-Hydroxypropin, -propen bzw. -propan in 17-Stellung erfolgt durch Umsetzung des 17-Ketons mit dem Dianion des Propargylalkohols (3-Hydroxypropin), zum Beispiel dem in situ generierten Dikaliumsalz des Propargylalkohols, zum 17α-(3-Hydroxyprop-1-inyl)-17β-hydroxyderivat oder mit metallierten Derivaten des 3-Hydroxypropins, zum Beispiel mit 1-Lithium-3-(tetrahydropyran2'-yloxy)-prop-1-in-1-id, zum 17-[3-(Tetrahydropyran-2'-yloxy)-prop-1-inyl]-17β-hydroxyderivat, die anschließend zu den 17-(3-Hydroxypropyl- bzw. Hydroxypropenyl)-17β-hydroxy-Verbindungen hydriert werden können. Das gelingt zum Beispiel durch Hydrierung bei Raumtemperatur und Normaldruck in Lösungsmitteln wie Methanol, Ethanol, Propanol, Tetrahydrofuran (THF) oder Essigester unter Zusatz von Edelmetall-Katalysatoren wie Platin oder Palladium.

Die Einführung homologer Hydroxyalkin-, Hydroxyalken- und Hydroxyalkangruppen erfolgt in entsprechender Weise mit Homologen des Propargylalkohols.

Die Verbindung mit der Z-konfigurierten Doppelbindung in der Hydroxypropenylgruppe entsteht durch Hydrieren der acetylenischen Dreifachbindung mit einem desaktivierten Edelmetallkatalysator (J. Fried, J.A. Edwards: Organic Reactions in Steroid Chemistry, Van Nostrand Reinhold Company 1972, Seite 134; und H.O. House: Modern Synthetic Reactions 1972, Seite 19).Als desaktivierte Edelmetallkatalysatoren kommen beispielsweise 10% Palladium auf Bariumsulfat in Gegenwart eines Amins oder 5 % Palladium auf Calciumcarbonat unter Zusatz von (Blei(II)acetat infrage. Die Hydrierung wird nach der Aufnahme von einem Äquivalent Wasserstoff abgebrochen.

Die Verbindung mit der E-konfigurierten Doppelbindung in der Hydroxypropenylgruppe entsteht durch Reduktion

der acetylenischen Dreifachbindung in an sich bekannter Weise. In der Literatur sind eine ganze Reihe von Methoden zur Umwandlung von Alkinen in trans-Olefine beschrieben, beispielsweise die Reduktion mit Natrium in flüssigem Ammoniak (J. Am. Chem. Soc. 63 (1941) 216), mit Natriumamid in flüssigem Ammoniak (J. Chem. Soc. 1955, 3558), mit Lithium in niedermolekularen Aminen (J. A. Chem. Soc. 77 (1955) 3378), mit Boranen (J. Am. Chem. Soc. 93 (1971) 3395 und 94 (1972) 6560), mit Diisobutylaluminiumhydrid und Methyl-Lithium (J. Am. Chem. Soc. 89 (1967) 5085) und insbesondere mit Lithiumaluminiumhydrid/Alkoholat (J. Am. Chem. Soc. 89 (1967) 4245). Eine weitere Möglichkeit ist die Reduktion der Dreifachbindung mit Chrom(II)-sulfat in Gegenwart von Wasser oder Dimethylformamid in schwach saurem Milieu (J. Am. Chem. Soc. 86 (1964) 4358) sowie allgemein die Reduktion durch Einwirkung von Übergangsmetallverbindungen unter Wechsel der Oxydationsstufe.

Die Einführung der Hydroxyalkene kann auch direkt erfolgen durch Addition einer entsprechenden metallierten Hydroxyalkenylverbindung, wie zum Beispiel 1-Lithium-3-(tetrahydropyran-2'-yloxy)-prop-1(E)-en (J. Org. Chem. 40 2265) oder 1-Lithium-3-(tetrahydropyran-2'-yloxy)-prop-1(Z)-en (Synthesis 1981, 999) Homologe können auf diese Art ebenfalls eingeführt werden.

Die Einführung von 3-Hydroxypropan in 17-Stellung kann ebenfalls direkt durch Umsetzung des 1.7-Ketons mit metallierten Derivaten von 3-Halogen-propanolen - wobei die Hydroxygruppe im Metallierungsschritt als Alkoholat (Tetrahedron Letters 1978, 3013) oder als geschützte Funktion (J. Org. Chem. 37, 1947) vorliegt - zu der 17-(3-Hydroxypropyl)-17β-hydroxy-verbindung bzw. zu der an der terminalen Hydroxygruppe geschützten Verbindung erfolgen. Als Schutzgruppen kommmen zum Beispiel die Ethoxyethyl-, Tetrahydropyranyl- und Methoxymethylgruppen in Frage.

Werden Endprodukte der Formel I gewünscht mit $R^5/R^6$ in der Bedeutung von

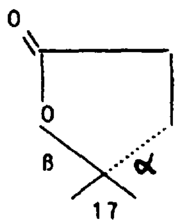

so wird die 17-(3-Hydroxypropyl)-Verbindung in an sich bekannter Weise oxydiert, zum Beispiel mit Jones' Reagenz, Braunstein, Pyridiniumdichromat, Pyridiniumchlorochromat, Chromsäure-Pyridin oder dem Fetizon-Reagenz Silbercarbonat/Celite (Compt. rend. 267 [1968]900).

Die Darstellung von Endprodukten der Formel I mit $R^5/R^6$ in der Bedeutung von

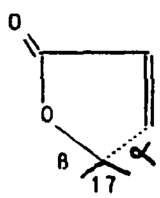

erfolgt durch Ringschlußreaktion des entsprechenden 17-(3-Hydroxyprop-1-(Z)enyl-17-β-hydroxy-Eduktes.

Sollen $R^5/R^6$ gemeinsam für

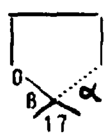

stehen, so wird entweder eine vorstehend beschriebene Dihydrofuranverbindung katalytisch hydriert oder die entsprechende 17-(3-Hydroxypropyl)-17β-hydroxy-verbindung cyclisiert.

Der Aufbau der 17-Cyanmethylseitenkette erfolgt in an sich bekannter Weise aus dem 17-Keton zum Beispiel über das 17-Spiroepoxid und Spaltung des Spiroepoxids mit HCN gemäß Z. Chem. 18 (1978) 259-260.

Auch die Einführung der 17-Hydroxyacetylseitenkette erfolgt nach an sich bekannten Methoden, beispielsweise nach den in J. Org. Chem. 47 (1982), 2993-2995, Chem.Ber. 113 (1984), 1184 bzw. US-Patent 4.600.538 beschriebenen Methoden.

Zur Einführung der Gruppierungen

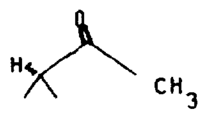

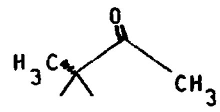

wird das 17-Keton mit Tosylmethylisocyanid (Chem. Ind. 1972 213) in die 17-Nitrilverbindung (Tetrahedron 31 (1975), 2151) überführt, das direkt mit Methyllithium oder Methylmagnesiumbromid in die 17-Acetylverbindung umgewandelt werden kann, welche nach Enolisierung mit K-tert.-Butylat in Tetrahydrofuran und Umsetzung mit Methyljodid die gewünschte 17α-Methyl-17βacylgruppierung liefert. Diese Sequenz Methyladdition an das Nitril und anschließende Alkylierung kann auch in umgekehrter Folge ausgeführt werden.

In Z bzw. im 19,11β-Phenylenring vorhandene freie Hydroxy- bzw. Hydroxy-, Mercapto- und/oder Aminogruppen können in an sich bekannter Weise alkyliert oder acyliert werden.

Im 19,11β-Phenylenring enthaltene Sulfide und/oder Dialkylamine können durch geeignete Oxidationsmittel (zum Beispiel Wasserstoffperoxid bzw. Persäuren) in die gewünschten Sulfoxide (n=1), N-oxide (n=1) [siehe z.B. Kontakte (Darmstadt) 1986, 3, S. 12] bzw. Sulfone (n=2) überführt werden.

Verbindungen mit einem Dialkylamin-Substituenten im 19,11β-Phenylenring können durch Reaktion mit Bromcyan in aprotischen Lösungsmitteln wie zum Beispiel Dioxan, Benzol oder Toluol bei erhöhter Temperatur (Amin-Abbau nach Braun) analog den zum Beispiel in Org. Reactions 7, 198 (1953), K.W. Bentley, Techniques of Organic Chemistry 11, 773 (1963) und Houben-Weyl, 5/4, 151 (1960) angegebenen Vorschriften in guter Ausbeute in die entsprechenden (N-Cyan-N-alkylaminoaryl)derivate überführt werden.

Diese werden je nach der letztlich gewünschten Bedeutung von $R^{12}$ im Endprodukt in an sich bekannter Weise zu den entsprechenden Dialkylamin-Verbindungen (zum Beispiel mit Diisobutylaluminiumhydrid in Toluol zu den N-Formyl-N-alkylaminophenyl-Zwischenprodukten und anschließend mit Lithiumaluminiumhydrid) bzw. N-H-N-alkyl-Verbindungen reduziert (zum Beispiel mit Lithiumaluminiumhydrid oder mit Lithium in flüssigem Ammoniak). Die letzteren werden anschließend gewünschtenfalls in literaturbekannter Weise acyliert und gegebenenfalls anschließend in bekannter Weise mit zum Beispiel Lithiumaluminiumhydrid zum neuen Dialkylaminderivat reduziert (s. DE 36 23 038).

Die erhaltenen Verbindungen der allgemeinen Formel I mit X in der Bedeutung eines Sauerstoffatoms können gewünschtenfalls durch Umsetzung mit Hydroxylaminhydrochlorid in Gegenwart von tertiären Aminen bei Temperaturen zwischen -20 und +40°C in die Oxime (Formel I mit X in der Bedeutung der Hydroxyiminogruppierung $\rangle N\sim OH$, wobei die Hydroxygruppe syn- oder antiständig sein kann) überführt werden. Geeignete tertiäre Basen sind beispielsweise Trimethylamin, Triäthylamin, Pyridin, N,N-Dimethylaminopyridin, 1,5-Diazabicyclol[4.3.0]nonen5 [DBN) und 1,5-Diazabicyclo[5.4.0]undecen-5 (DBU), wobei Pyridin bevorzugt ist.

Die neuen Verbindungen der allgemeinen Formel I sowie deren pharmazeutisch verträgliche Additionssalze mit Säuren sind wertvolle Pharmaka. So verfügen sie über eine starke Affinität zum Gestagenrezeptor und besitzen einen überraschend großen Bereich an gestagenen, antigestagenen, antiglucocorticoiden, antimineralcorticoiden und antiandrogenen Eigenschaften. Diese wichtigen biologischen Wirksamkeiten können für medizinische Zwecke genutzt werden.

Die starke Affinität zum Gestagenrezeptor ergibt sich aus dem bekannten, u.a. in der EP-A 0190 759 beschriebenen Gestagenrezeptor-Bindungstest. Demnach besitzen die erfindungsgemäßen Verbindungen 11β,19-[4-(4-Cyanphenyl)-o-phenylen]-17β-hydroxy-17α-(3-hydroxyprop-1(Z)enyl)-4,8-androstadien-3 -on (A) sowie 11β,19-[4-(4-Cyanphenyl)-o-phenylen]-17α-ethinyl-17β-hydroxy-4,8-androstadien-3-on (B) K-Werte von 1 bzw. 0,7, die Beleg für eine starke Bindung an den Gestagenrezeptor sind.

Gegenüber den in der EP-A 0 283 428 beschriebenen Verbindungen, die keine 8,9-Doppelbindung aufweisen, zeichnen sich vorliegende Verbindungen durch eine stärkere Bindung an den Gestagen-Rezeptor aus: die entsprechenden, zu Verbindung A und B analogen Verbindungen, ohne 8,9-Doppelbindung besitzen lediglich K-Werte von 7,5 bzw. 5,6.

Zur Kennzeichnung der antigestagenen Wirkung wurde die abortive Wirkung an graviden Ratten nach dem in der EP-A 0 283 428 beschriebenen Test bestimmt. Verbindung A ist demnach noch bei einer Dosis von 0,1 mg s.c. voll und die Verbindung B bei einer Dosis von 0,3 mg s.c. zu 75% abortiv wirksam.

Wirkstoffe dieser Art mit ausgeprägter antigestagener Aktivität sind zur Auslösung von Aborten geeignet, da sie das zur Aufrechterhaltung der Schwangerschaft erforderliche Progesteron vom Rezeptor verdrängen. Sie sind deshalb wertvoll und interessant im Hinblick auf ihre Verwendung zur Fertilitätskontrolle der Frau.

Sie können auch gegen hormonelle Unregelmäßigkeiten, zur Menstruationsauslösung und zur Geburtseinleitung eingesetzt werden.

Außerdem können sie für die Behandlung von hormonabhängigen Carcinomen eingesetzt werden.

Die erfindungsgemäßen Verbindungen der allgemeinen Formel I sowie deren pharmazeutisch verträgliche Additionssalze mit Säuren weisen auch eine antiglucocorticoide Aktivität auf und können somit auch als Arzneimittel zur Therapie corticoid-induzierter Störungen (Glaukom) sowie zur Bekämpfung von Nebenwirkungen, die bei langfristiger Behandlung mit Glucocorticoiden auftreten (Cushing-Syndrom) eingesetzt werden. Sie ermöglichen daher auch die auf eine Supersekretion der Glucocorticoide zurückzuführenden Störungen, vor allem die Adipositas, Arteriosklerose, Hypertension, Osteoporose, Diabetes sowie die Insomnie zu bekämpfen.

Die erfindungsgemäßen Verbindungen der allgemeinen Formel I sowie deren pharmazeutisch verträgliche Additionssalze mit Säuren mit gestagener Aktivität können beispielsweise bei der Therapie von Amenorrhoe, Dysmenorrhoe, Hypermenorrhoe und lutealer Insuffizienz, solche mit antimineralcorticoiden Eigenschaften zur Behandlung von Krankheitszuständen, an denen ein Hyperaldosteronismus beteiligt ist, verwendet werden.

Die Erfindung betrifft somit auch Arzneimittel auf Basis der pharmazeutisch verträglichen, d.h. in den verwendeten Dosen nicht toxischen Verbindungen der allgemeinen Formel I sowie deren pharmazeutisch verträgliche Additionssalze mit Säuren, gegebenenfalls zusammen mit den üblichen Hilfs-und Trägerstoffen.

Die erfindungsgemäßen Verbindungen und deren Salze können nach an sich bekannten Methoden der Galenik zu pharmazeutischen Präparaten für die enterale, perkutane, parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen, Salben, Cremes und Gelen verabreicht werden.

Der oder die Wirkstoffe können dabei mit den in der Galenik üblichen Hilfsstoffen wie z.B. Gummiarabikum, Talk, Stärke, Mannit, Methylcellulose, Laktose, Tensiden wie Tweens(R) oder Myrj(R), Magnesiumstearat, wäßrigen oder nichtwäßrigen Trägern, Paraffinderivaten, Netz-, Dispergier-, Emulgier-,-Konservierungsmitteln und Aromastoffen zur Geschmackskorrektur (z.B. ätherischen Ölen) gemischt werden.

Die Erfindung betrifft somit auch pharmazeutische Zusammensetzungen, die als Wirkstoff zumindest eine erfindungsgemäße Verbindung oder eines ihrer Additionssalze mit pharmazeutisch verträglichen Säuren enthalten. Als Additionssalze der erfindungsgemäßen Produkte mit Säuren sind insbesondere die Hydrochloride und die Methansulfonate zu nennen. Eine Dosiseinheit enthält etwa 1-100 mg Wirkstoff(e).

Die Dosierung der erfindungsgemäßen Verbindungen liegt beim Menschen bei etwa 0,1-1000 mg pro Tag, vorzugsweise 0,1 - 300 mg pro Tag.

Die folgenden Beispiele erläutern die Herstellung der erfindungsgemäßen Verbindungen.

Die Herstellung der erfindungsgemäß benötigten Ausgangsverbindungen erfolgt gemäß dem nachfolgenden Schema:

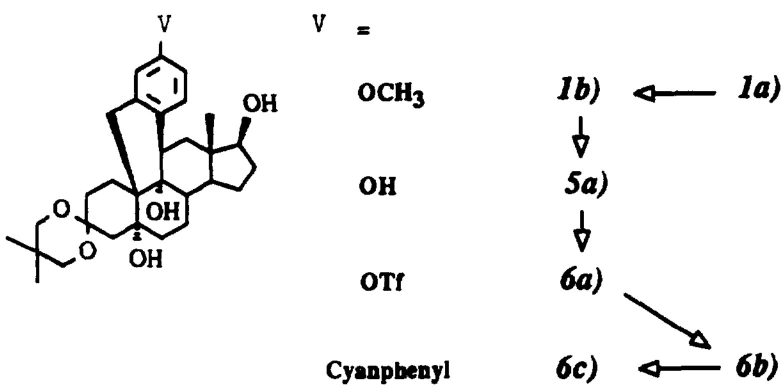


(OTf = $OSO_2CF_3$)

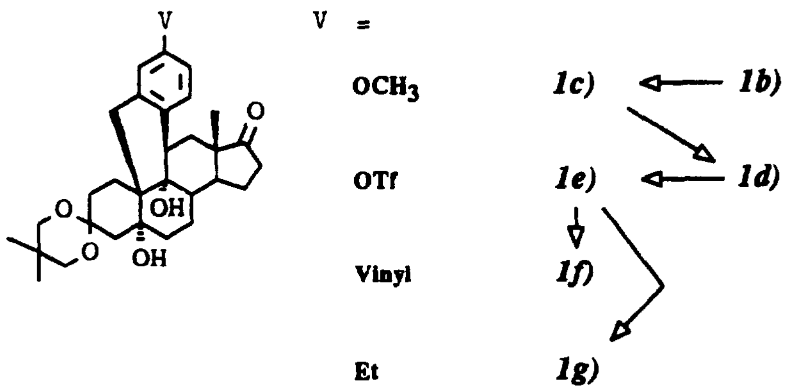


Cyanphenyl erfolgt *aus 6c)* ***Oxidation analog 1c)***

Übersichtsartikel zu Kupplungen : J. Organometallic Chem **392** (1990)285 Die physikalischen Daten dieser Ausgangsverbindungen befinden sich in Tabelle 1.
Vorschriften zur Herstellung der Ausgangsverbindungen:

1) 9α, 17β-Dihydroxy-11β, 19-(4-ethyl-o-phenylen)-17-(prop-1-inyl)-4-androsten-3-on

a) 19-(2-Brom-5-methoxyphenyl)-3,3-(2,2-dimethyltrimethylendioxy)-9α, 11α-epoxy-androstan-5α, 17β-diol

Zu einer Lösung von 47,6 g (82,6 mmol) 19-(2-Brom-5-methoxyphenyl)-3,3-(2,2dimethyltrimethylendioxy)-9,11-androsten-5α,17β-diol (EP-A 0283428) in 0,8 1 Methylenchlorid werden bei Raumtemperatur nacheinander 225 ml einer 0.5 m wäßrigen Natriumhydrogencarbonatlösung und 22,6 g 67%ige m-Chlorperbenzoesäure zugegeben. Anschließend wird das Reaktionsgemisch 1,5 Stunden nachgerührt. Zur Aufarbeitung trennt man die wäßrige Phase ab, extrahiert sie mit wenig Methylenchlorid und vereinigt die organischen Phasen. Diese werden nacheinander mit gesättigter Natriumthiosulfatlösung, 5%iger Natronlauge und Wasser gewaschen, über Natriumsulfat getrocknet und am Vakuum eingeengt. Die Reinheit des Rohprodukts (47,4 g) ist ausreichend für die weitere Umsetzung unter b). Zur Charakterisierung werden 400 mg des Rohprodukts an Kieselgel mit einem Gemisch aus Ethylacetat/Hexan chromatographiert. Man erhält 357 mg 19-(2-Brom-5-methoxyphenyl)-3,3-(2,2-dimethyltrimethylendioxy)-9α,11α-epoxyandrostan-5α,17β-diol.

b) 3,3-(2,2-Dimethyltrimethylendioxy)-11β, 19-(4-methoxy-o-phenylen)-androstan-5α, 9α, 17β-triol

41,4 g (70 mmol) 19-(2-Brom-5-methoxyphenyl)-3,3-(2,2-dimethyltrimethylendioxy)-9α,11α-epoxy-androstan-5α, 17β-diol, gelöst in 350 ml absolutem Diethylether, werden unter Schutzgas bei Raumtemperatur zu 435 ml einer 0,8 etherischen Methylmagnesiumjodidlösung zugegeben. Nach 30-minütigem Nachrühren wird das Reaktionsgemisch mit 825 ml einer 1,6 m n-Butyllithiumlösung (Hexan) versetzt und über Nacht nachgerührt. Anschließend wird es vorsichtig auf eisgekühlte gesättigte wäßrige Ammoniumchloridlösung gegossen, die organische Phase abgetrennt und die wäßrige mit Ethylacetat nachextrahiert. Die organischen Phasen werden vereinigt, mit gesättigter, wäßriger Kochsalzlösung gewaschen, über Natriumsulfat getrocknet und am Vakuum eingeengt. Das Rohprodukt (37,2 g) wird an Kieselgel mit einem Gemisch aus Ethylacetat/Hexan chromatographiert und man erhält 31,3 g der Titelverbindung als weißen Schaum. Fp.: = 254-255°C (Ethylacetat)

c) 5α, 9α-Dihydroxy-3,3-(2,2-dimethyltrimethylendioxy)-11β, 19-(4-methoxy-o-phen-ylen)-androstan-17-on

Zu einem Gemisch aus 120 ml Pyridin und 875 ml Methylenchlorid werden bei 0°C portionsweise 35,1 g Chromtrioxid zugegeben. Anschließend werden 30 g 3,3-(2,2-Dimethyltrimethylendioxy)-11β,19-(4-methoxy-o-phenylen)-androstan-5α,9α,17β-triol, gelöst in 100 ml Methylenchlorid, langsam bei der gleichen Temperatur zugetropft und das Reaktionsgemisch weitere 1,5 Stunden bei Eisbadtemperatur nachgerührt. Nach Beendigung des Rührens läßt man die festen Bestandteile sich absetzen, dekantiert die überstehende Phase ab und wäscht den Niederschlag mehrmals gründlich mit Methylenchlorid aus. Die vereinigten organischen Phasen werden durch Waschen mit 0,5 m Kaliumhydroxidlösung von restlichen anorganischen Bestandteilen befreit, mit Wasser neutral gewaschen, über Natriumsulfat

getrocknet und am Vakuum eingeengt. Durch Chromatographie des Rückstandes an Kieselgel mit einem Gemisch aus Ethylacetat/Hexan werden 26,7 g der Titelverbindung als weißer Schaum isoliert.

d) 5α, 9α-Dihydroxy-3,3-(2,2-dimethyltrimethylendioxy)-11β, 19-(4-hydroxy-o-phenylen)-androstan-17-on

25,5 g (50 mml) 5α,9α-Dihydroxy-3,3-(2,2-dimethyltrimethylendioxy)-11β,19-(4-methoxy-o-phenylen)-androstan-17-on werden in 250 ml absolutem Dimethylformamid gelöst und unter Schutzgas mit 14 g Natriumthiomethylat versetzt. Unter Inertgasatmosphäre wird das Reaktionsgemisch 3 Stunden zum Rückfluß erhitzt, anschließend wird auf Raumtemperatur abgekühlt und dann auf 4 l Eiswasser gegossen. Es wird solange nachgerührt, bis das Rohprodukt als weißer Feststoff ausgeflockt ist. Anschließend wird es abgesaugt, mit viel Wasser gewaschen und am Vakuum getrocknet. Es werden 22,6 g der Titelverbindung als Rohprodukt erhalten, dessen Reinheit für die nachfolgenden Umsetzungen ausreichend ist.

e) 5α, 9α-Dihydroxy-3,3-(2,2-dimethyltrimethylendioxy)-11β, 19-(4-trifluormethan-sulfonyloxy-o-phenylen)-androstan-17-on

21,85 g (44 mmol) 5α,9α-Dihydroxy-3,3-(2,2-dimethyltrimethylendioxy)-11β,19(4-hydroxy-o-phenylen)-androstan-17-on werden in 675 ml absolutem Methylenchlorid gelöst und mit 29,8 g 4-Dimethylaminopyridin versetzt. Unter Schutzgas wird die Lösung anschließend auf -70°C gekühlt und durch langsames Zutropfen mit 9,7 ml Trifluormethansulfonsäureanhydrid gelöst in 60 ml absolutem Methylenchlorid versetzt. Nach 30-minütigem Nachrühren bei -70°C wird das Reaktionsgemisch auf gesättigte wäßrige Natriumhydrogencarbonatlösung gegossen, die organische Phase abgetrennt und die wäßrige Phase mehrmals mit Methylenchlorid extrahiert. Die vereinigten organischen Phasen werden mit gesättigter Natriumchloridlösung gewaschen, über Natriumsulfat getrocknet und am Vakuum eingeengt. Man erhält nach Chromatographie des Rückstandes an Kieselgel mit einem Gemisch aus Ethylacetat/Hexan 23,1 g der Titelverbindung als weißen Schaum.

f) 5α, 9α-Dihydroxy-3,3-(2,2-dimethyltrimethylendioxy)-11β, 19-(4-vinyl-o-phenylen)-androstan-17-on

4 g 5α,9α-Dihydroxy-3,3-(2,2-dimethyltrimethylendioxy)-11β,19-(4-trifluor-methansulfonyloxy-o-phenylen)-androstan-17-on werden in 64 ml absolutem Dimethylformamid gelöst und mit 542 mg Lithiumchlorid, 0,37 g Tetrakistriphenylpalladium und 2,34 ml Tributylvinylzinn versetzt. Anschließend wird das Reaktionsgemisch 1,5 Stunden bei 110°C unter Schutzgas gerührt und dann auf Raumtemperatur abgekühlt. Nach Filtration über Celite und Waschen des Filterrückstandes mit Ethylacetat wird die organische Phase mit gesättigter Natriumchloridlösung gewaschen, über Natriumsulfat getrocknet und am Vakuum eingeengt. Chromatographie des Rückstandes an Kieselgel mit einem Gemisch aus Ethylacetat/Hexan ergibt 2,96 g der Titelverbindung als weißen Schaum.

g) 5α, 9α-Dihydroxy-3,3-(2,2-dimethyltrimethylendioxy)-11β, 19-(4-ethyl-o-phenylen)-androstan-17-on

1,5 g 5α,9α-Dihydroxy-3,3-(2,2-dimethyltrimethylendioxy)-11β,19-(4-vinylo-phenylen)-androstan-17-on werden in 15 ml absolutem Tetrahydrofuran gelöst und nach Zugabe von 1,5 ml Pyridin mit 150 mg Palladium auf Bariumsulfat (10%ig) als Katalysator bei Normaldruck hydriert. Nach Aufnahme eines Äquivalents Wasserstoff wird das Reaktionsgemisch über Celite abgesaugt, der Filterrückstand mit Essigester nachgewaschen und das Filtrat am Vakuum eingeengt. Kristallisation des Rohproduktes aus Ethylacetat führt zu 1,26 g der Titelverbindung.

h) 3,3-(2,2-Dimethyltrimethylendioxy)-11β, 19-(4-ethyl-o-phenylen)-17-(prop-1-inyl)-androstan-5α, 9α, 17β-triol

30 ml absolutes Tetrahydrofuran werden bei 0°C mit Propin gesättigt. Anschließend werden zu dieser Lösung 3,7 ml einer 1,6 m-Butyllithiumlösung (Hexan) langsam ohne größere Temperaturerhöhung zugetropft. Nach 15-minütigem Nachrühren tropft man bei Eisbadkühlung langsam eine Lösung von 300 mg 5α,9α-Dihydroxy-3,3-(2,2-dimethyltrimethylendioxy)-11β,19-(4-ethylo-phenylen)-androstan-17-on, gelöst in 6 ml absolutem Tetrahydrofuran, zu dieser Reaktionsmischung und läßt sie über Nacht nachrühren. Danach wird das Reaktionsgemisch auf Wasser gegossen, die wäßrige Phase mit Ethylacetat extrahiert und die organische Phase mit Natriumchloridlösung gewaschen. Nach Trocknen über Natriumsulfat und Einengen der organischen Phase am Vakuum wird der Rückstand an Aluminiumoxid (neutral, Stufe III) chromatographiert. Es werden 295 mg der Titelverbindung als weißer Schaum erhalten.

i) 9α, 17β-Dihydroxy-11β, 19-(4-ethyl-o-phenylen)-17-(prop-1-inyl)-4-androsten-3-on

280 mg 3,3-(2,2-Dimethyltrimethylendioxy)-11β,19-(4-ethyl-o-phenylen)-17-(prop-1-inyl)-androstan-5α,9α,17β-triol werden in 20 ml Aceton gelöst und mit 0,1 ml 4 n wäßriger Salzsäure versetzt. Nach 3-stündigem Nachrühren bei 40°C wird das Reaktionsgemisch auf gesättigte Natriumhydrogencarbonatlösung gegossen und die wäßrige Phase mit Methylenchlorid extrahiert. Die vereinigten organischen Phasen werden mit gesättigter Natriumchloridlösung gewaschen, über Natriumsulfat getrocknet und am Vakuum eingeengt. Man erhält nach Chromatographie des Rückstandes an Kieselgel mit einem Gemisch aus Ethylacetat/Hexan 234 mg der Titelverbindung als weißen Schaum.
Fp.: = 155-160°C (Hexan/Methylenchlorid)

2) <u>9α, 17β-Dihydroxy-11β, 19-(4-vinyl-o-phenylen)-17-(prop-1-inyl)-4-androsten-3-on</u>

a) 3,3-(2,2-Dimethyltrimethylendioxy)-11β, 19-(4-vinyl-o-phenylen)-17-(prop-1-inyl)-androstan-5α, 9α, 11β-triol

Analog der unter 1h) beschriebenen Vorschrift werden 1 g der unter f hergestellten Ketoverbindung in die entsprechende 17-Propinylverbindung überführt. Es werden 0,97 g der obigen Verbindung als weißer Schaum erhalten.

b) 9α, 17β-Dihydroxy-11β, 19-(4-vinyl-o-phenylen)-17-(prop-1-inyl)-4-androsten-3-on

Analog der unter 1i) beschriebenen Vorschrift werden 0,9 g der unter d) hergestellten Propinylverbindung umgesetzt. Es werden 660 mg der Titelverbindung als weißer Schaum erhalten.
Fp.: = 171-175°C (Diisopropylether/Methylenchlorid)

3) <u>9α, 17β-Dihydroxy-11β, 19-[4-(3-pyridyl)-o-phenylen]-17-(prop-1-inyl)-4-androsten-3-on</u>

a) 5α, 9α-Dihydroxy-3,3-(2,2-dimethyltrimethylendioxy)-11β, 19-(4-tri-n-butylstannyl-o-phenylen)-androstan-17-on -

2,6 g des unter le) hergestellten Triflats werden in 41 ml absolutem Dioxan gelöst und unter Schutzgas mit 6,2 ml Hexa-n-butylzinn, 521 mg Lithiumchlorid und 190 mg Tetrakistriphenylphosphinpalladium versetzt. Anschließend wird das Reaktionsgemisch auf 110°C erwärmt, 1 Stunde nachgerührt, auf Raumtemperatur abgekühlt und am Vakuum eingeengt. Der Rückstand wird an Kieselgel mit einem Gemisch aus Ethylacetat/Hexan chromatographiert. Man erhält 2,75 g der obigen Verbindung als weißen Schaum.

b) 5α, 9α-Dihydroxy-3,3-(2,2-dimethyltrimethylendioxy)-11β, 19-[4-(3-pyridyl)-o-phenylen]-androstan-17-on

2,7 g des unter a) hergestellten Zinnorganyls werden in 41 ml absolutem Toluol gelöst, mit 190 mg Tetrakistriphenylphosphinpalladium und 4 ml 3-Brompyridin versetzt. Anschließend wird das Reaktionsgemisch 17 Stunden auf 110°C erwärmt, auf Raumtemperatur abgekühlt und am Vakuum eingeengt. Der Rückstand wird an Kieselgel mit einem Gemisch aus Ethylacetat/Hexan chromatographiert. Man erhält 1,47 g der obigen Verbindung als weißen Schaum.

c) 3,3-(2,2-Dimethyltrimethylendioxy)-11β, 19-[4-(3-pyridyl-o-phenylen]-17-(prop -1inyl)-androstan-5α, 9α, 17β-triol

Analog der unter 1h) beschriebenen Vorschrift werden 400 mg der unter b) hergestellten Ketoverbindung in die entsprechende 17-Propinylverbindung überführt. Es werden 402 mg der obigen Verbindung als weißer Schaum erhalten.

d) 9α, 17β-Dihydroxy-11β, 19-[4-(3-pyridyl)-o-phenylen]-17-(prop-1-inyl)-4-andro-sten-3-on

Analog der unter 1i) beschriebenen Vorschrift werden 380 mg der unter c) hergestellten Propinylverbindung umgesetzt. Es werden 288 mg der Titelverbindung als weißer Schaum erhalten.
Fp.: = 203-205°C (Diisopropylether)

4) <u>9α, 17β-Dihydroxy-11β, 19-(4-methoxy-o-phenylen)-4-androsten-3-on</u>

Analog der unter 1i) beschriebenen Vorschrift werden 0,5 g der unter 1b) hergestellten Hydroxyverbindung umgesetzt. Es werden 306 mg der Titelverbindung als weißer Schaum erhalten. Fp.: = 175-177°C (Ethylacetat)

5) <u>9α, 17β-Dihydroxy-11β, 19-(4-hydroxy-o-phenylen)-4-androsten-3-on</u>

a) 3,3-(2,2-Dimethyltrimethylendioxy)-11β, 19-(4-hydroxy-o-phenylen)-androstan-5α, 9α, 17β-triol

Analog der unter 1d) beschriebenen Vorschrift werden 2,5 g der unter 1b) hergestellten Methoxyverbindung umgesetzt. Es werden nach Chromatographie an Kieselgel mit einem Gemisch aus Ethylacetat/Hexan 2,15 g Phenol als weißer Schaum erhalten.

b) 9α, 17β-Dihydroxy-11β, 19-(4-hydroxy-o-phenylen)-4-androsten-3-on

Analog der unter 1i) beschriebenen Vorschrift werden 750 mg des unter a) hergestellten Phenols umgesetzt. Es werden 394 mg der Titelverbindung als weißer Schaum erhalten.
Fp.: = 146-148°C (Ethylacetat)

6) <u>9α, 17β-Dihydroxy-11β, 19-[4-(4-cyanophenyl)-o-phenylen]-4-androsten-3-on</u>

a) 3,3-(2,2-Dimethyltrimethylendioxy)-11β, 19-(4-trifluormethansulfonyloxy o-phenylen)-androstan-5α, 9α, 17β-triol

Analog der unter 1e) beschriebenen Vorschrift werden 1,25 g des unter 5a) hergestellten Phenols umgesetzt. Es werden nach Chromatographie an Kieselgel mit einem Gemisch aus Ethylacetat/Hexan 1,3 g Triflat als weißer Schaum erhalten.

b) 3,3-(2,2-Dimethyltrimethylendioxy)-11β, 19-(4-tri-n-butylstannyl-o-phenylen)-androstan-5α, 9α, 17β-triol

Analog der unter 3a) beschriebenen Vorschrift werden 1,25 g des unter a) hergestellten Triflats umgesetzt. Es werden nach Chromatographie an Kieselgel mit einem Gemisch aus Ethylacetat/Hexan 1,53 g Zinnorganyl als weißer Schaum erhalten.

c) 3,3-(2,2-Dimethyltrimethylendioxy)-11β, 19-[4-(4-cyanophenyl)-o-phenylenl]-androstan-5α, 9α, 17β-triol

Analog der unter 3b) beschriebenen Vorschrift werden 1,5 g des unter b) hergestellten Zinnorganyls mit 4 g 4-Brombenzonitril umgesetzt. Es werden nach Chromatographie an Kieselgel mit einem Gemisch aus Ethylacetat/ Hexan 0,73 g Kupplungsprodukt als weißer Schaum erhalten.

d) 9α, 17β-Dihydroxy-11β, 19-[4-(4-cyanophenyl)-o-phenylen]-4-androsten3-on

Analog der unter 1i) beschriebenen Vorschrift werden 700 mg des unter c) hergestellten Benzonitrils umgesetzt. Es werden 512 mg der Titelverbindung als weißer Schaum erhalten.
Fp.: = 186-190°C (Diisopropylether)

**Beispiel 1**

**<u>17β-Hydroxy-11β,19-(4-methoxy-o-phenylen)-17α-(prop-1-inyl)-4,8-androstadien-3-on</u>**

a) 9α-Hydroxy-11β,19-(4-methoxy-o-phenylen)-4-androsten-3,17-dion

2,75 g 5α,9α-Dihydroxy-3,3-(2,2-dimethyltrimethylendioxy)-11β,19-(4-methoxy-o-phenylen)-androstan-17-on werden in 150 ml Aceton gelöst und unter Schutzgas mit 7,5 ml 4 n wäßriger Salzsäure versetzt. Nach 3-stündigem Rühren bei 40°C wird das Reaktionsgemisch auf kalte gesättigte Natriumhydrogencarbonatlösung gegossen und die wäßrige Phase mehrmals mit Methylenchlorid extrahiert. Die vereinigten organischen Phasen werden über Natriumsulfat getrocknet und am Vakuum eingeengt. Der Rückstand wird an Kieselgel mit einem Gemisch aus Ethylacetat/ Hexan chromatographiert Man erhält 1,96 g 9α-Hydroxy-11β,19-(4-methoxy-o-phenylen)-4-androsten-3,17-dion als weißen Schaum.
Fp. = 114 °C (Ethylacetat)

b)11β,19-(4-Methoxy-o-phenylen)-4,8-androstadien-3,17-dion

1,8 g 9α-Hydroxy-11β,19-(4-methoxy-o-phenylen)-4-androsten-3,17-dion werden in 21 ml absolutem Pyridin gelöst und langsam bei 0 °C mit 0,31 ml Thionylchlorid versetzt. Nach 30-minütigem Nachrühren wird das Reaktionsgemisch vorsichtig in gesättigte Natriumhydrogencarbonatlösung eingerührt und die wäßrige Phase mehrmals mit Essigester extrahiert. Die vereinigten organischen Phasen werden mit gesättigter Natriumchloridlösung gewaschen, über Natriumsulfat getrocknet und am Vakuum eingeengt. Der Rückstand wird an Kieselgel mit einem Gemisch aus Ethylacetat/Hexan chromatographiert. Man erhält 1,26 g 11β,19-(4-Methoxy-o-phenylen)-4,8-androstadien-3,17-dion als weißen Schaum.

c) 3-Ethoxy-11β,19-(4-methoxy-o-phenylen)-3,5,8-androstatrien-17-on

1 g 11β,19-(4-Methoxy-o-phenylen)-4,8-androstadien-3,17-dion werden in 26 ml absolutem Methylenchlorid gelöst und bei 0 °C nacheinander mit 2,6 ml Triethylorthoformiat, 0,17 ml absolutem Ethanol und 14 mg para-Toluolsulfonsäure versetzt Nach 4-stündigem Nachrühren wird dem Reaktionsgemisch Triethylamin (0,1 ml) zugesetzt, die Reaktionslösung anschließend in gesättigte Natriumhydrogencarbonatlösung eingerührt und die wäßrige Phase mehrmals mit Methylenchlorid extrahiert Die vereinigten organischen Phasen werden mit gesättigter Natriumchloridlösung gewaschen, über Natriumsulfat getrocknet und am Vakuum eingeengt Der Rückstand wird direkt in den nachfolgenden Reaktionsschritt eingesetzt.

d) 3-Ethoxy-11β,19-(4-methoxy-o-phenylen)-17α-(prop-1-inyl)-3,5,8-androstatrien-17β-ol

120 ml absolutes Tetrahydrofuran werden bei 0 °C mit Propin gesättigt. Anschließend werden zu dieser Lösung 16 ml einer 1,6 m n-Butyllithiumlösung (Hexan) langsam ohne größere Temperaturerhöhung zugetropft. Nach 15-minütigem Nachrühren tropft man bei Eisbadkühlung langsam eine Lösung des unter c) dargestellten Ketons, gelöst in 25 ml absolutem Tetrahydrofuran, zu dieser Reaktionsmischung und läßt sie 60 Minuten nachrühren. Danach wird das Reaktionsgemisch auf Wasser gegossen, die wäßrige Phase mit Ethylacetat extrahiert und die organische Phase mit Natriumchloridlösung gewaschen. Nach Trocknen über Natriumsulfat und Einengen der organischen Phase am Vakuum wird der Rückstand, wie in der nachfolgenden Vorschrift beschrieben, direkt weiter umgesetzt.

e)17β-Hydroxy-11β,19-(4-methoxy-o-phenylen)-17α(prop-1-inyl)4,8-androstadien-3-on

Der unter d) erhaltene Rückstand wird analog der unter a) beschriebenen Vorschrift zur Titelverbindung umgesetzt. Nach Chromatographie an Kieselgel mit einem Gemisch aus Ethylacetat/Hexan werden 332 mg der Titelverbindung als gelblicher Schaum isoliert.
Fp.: 250-253 °C (Ethylacetat)
$[\alpha]^{20}_D$ = +70° ($CHCl_3$;c=0.525)

**Beispiel 2**

**11β,19-(4-Ethyl-o-phenylen)-17β-hydroxy-17α-(prop-1-inyl)-4,8-androstadien-3-on**

**A)** In analoger Sequenz der unter Beispiel 1 beschriebenen Reaktionsvorschriften werden ausgehend von 2,2 g 11β,19-(4-Ethyl-o-phenylen)-5α,9α-dihydroxy-3,3-(2'2-dimethyltrimethylendioxy)-androstan-17-on über a) 11β,19-(4-Ethyl-o-phenylen)-9α-hydroxy-4-androsten-3,17-dion, b) 11β,19-(4-Ethyl-o-phenylen)-4,8-androstadien-3,17-dion, c) 3-Ethoxy-11β,19-(4-ethyl-o-phenylen)-3,5,8-androstatrien-17-on und d) 3-Ethoxy-11β,19-(4-ethyl-o-phenylen)-17α-(prop-1-inyl)-3,5,8-androsstatrien-17β-ol 296 mg der Titelverbindung als gelblicher Schaum darmstellt.

$^1$H-NMR ($CDCl_3$) [δ] ppm: 7,27 (1H, d J=8,5Hz); 7,05 (1H, dd $J_1$=8,5 und $J_2$=1,5Hz); 6,84 (1H, d J=1,5Hz); 5,89 (1H,s); 3,73 (1H, d breit J=7,5Hz); 3,12 (1H, d J=17,5Hz); 2,94 (1H, d J=17,5Hz); 1,22 (3H, tr J=7,5Hz); 0,54 (3H, s)
$[\alpha]^{20}_D$ = + 74° ($CHCl_3$;c=0.465)

**B)** Alternativ können ausgehend von 1,3 g 11β,19-(4-Ethyl-o-phenylen)-4,8-androstadien-3,17-dion über selektive Ketalisierung zum 11β,19-(4-Ethyl-o-phenylen)-3,3-(2,2-dimethyltrimethylendioxy)-5,8-androstadien-17-on [1], Propinaddition zum 11β,19-(4-Ethyl-o-phenylen)-3,3-(2,2-dimethyltrimethylendioxy)-17α-(prop-1-inyl)-5,8-androstadien-17β-ol analog zur Vorschrift 1 d) und saurer Spaltung analog der Vorschrift 1 a) 325 mg der Titelverbindung dargestellt werden.

1,3 g 11β,19-(4-Ethyl-o-phenylen)-4,8-androstadien-3,17-dion werden in 85 ml absolutem Methylenchlorid gelöst und nacheinander mit 0,9 ml Trimethylorthoformiat, 1,8 g 2,2-Dimethyl-1,3-propandiol und 50 mg para-Toluolsulfonsäure versetzt. Nach 4-stündigem Nachrühren wird dem Reaktionsgemisch Triethylamin (0,5 ml) zugesetzt, die Reaktionslösung anschließend in gesättigte Natriumhydrogencarbonatlösung eingerührt und die wäßrige Phase mehrmals mit Methylenchlorid extrahiert. Die vereinigten organischen Phasen werden mit gesättigter Natriumchloridlösung

[1], 11β,19-(4-Ethyl-o-phenylen)-3,3-(2,2-dimethyltrimethylendioxy)-5,8-androstadien-17-on

gewaschen, über Natriumsulfat getrocknet und am Vakuum eingeengt. Der Rückstand wird an Kieselgel mit einem Gemisch aus Ethylacetat/Hexan chomatographiert. Man erhält 778 mg 11β,19-(4-Ethyl-o-phenylen)-3,3-(2,2-dimethyltrimethylendioxy)-5,8-androstadien-17-on als weißen Schaum.
Fp. = 145-147 °C (Diisopropylether)

**Beispiel 3**

**11β,19-[4-(4-Cyanphenyl)-o-phenylen]-17β-hydroxy-17α-(prop-1-inyl)-4,8-androstadien-3-on**

In analoger Sequenz der unter Beispiel 1 beschriebenen Reaktionsvorschriften werden ausgehend von 2,7 g 11β, 19-[4-(4-Cyanphenyl)-o-phenylen]-5α,9α-dihydroxy-3,3-(2,2-dimethyltrimethylendioxy)-androstan-17-on über a) 11β, 19-[4-(4-Cyanphenyl)-o-phenylen]-9α-hydroxy-4-androsten-3,17-dion, b) 11β,19-[4-(4-Cyanphenyl)-o-phenylen]-4,8-androstadien-3,17-dion, c) 11β,19-[4-(4-Cyanphenyl)-o-phenylen]-3-ethoxy-3,5,8-androstatrien-17-on und d) 11β, 19-[4-(4-Cyanphenyl)-o-phenylen]-3-ethoxy-17α-(prop-1-inyl)-3,5,8-androstatrien-17β-ol 312 mg der Titelverbindung als gelblicher Schaum dargestellt.
Fp.: 209-211°C (Methylenchlorid/Diisopropylether)
$[\alpha]^{20}_{D}$ = +94° ($CHCl_3$;c=0.505)

**Beispiel 4**

**11β,19-[4-(4-Cyanphenyl)-o-phenylen]-17α-ethinyl-17β-hydroxy-4,8-androstadien-3-on**

In analoger Sequenz der unter Beispiel 1 beschriebenen Reaktionsvorschriften werden ausgehend von 2,7 g 11β, 19-[4-(4-Cyanphenyl)-o-phenylen]-5α,9α-dihydroxy-3,3-(2,2-dimethyltrimethylendioxy)-androstan-17-on über a) 11β, 19-[4(4-Cyanphenyl)-o-phenylen]-9α-hydroxy-4-androsten-3,17-dion, b) 11β,19-[4-(4-Cyanphenyl)-o-phenylen] 4,8-androstadien-3,17-dion, c) 11β,19-[4-(4-Cyanphenyl)-o-phenylen]-3-ethoxy-3,5,8-androstatrien-17-on und d) 11β, 19-[4-(4-Cyanphenyl)-o-phenylen]-17α-ethinyl-3-ethoxy-3,5,8-androstatrien-17β-ol 341 mg der Titelverbindung als gelblicher Schaum dargestellt.
Fp.: 212-215°C (Ethylacetat)
$[\alpha]^{20}_{D}$ = + 155° ($CHCl_3$;c=0.515)

**Beispiel 5**

**11β,19-[4-(4-Cyanphenyl)-o-phenylen]-17β-hydroxy-17α-(3-hydroxyprop-1-inyl)-4,8-androstadien-3-on**

**A)** In analoger Sequenz der unter Beispiel 1 beschriebenen Reaktionsvorschriften werden ausgehend von 5,7 g 11β,19-[4-(4-Cyanphenyl)-o-phenylen]-5α,9α-dihydroxy-3,3-(2,2-dimethyltrimethylendioxy)-androstan-17-on über a) 11β,19-[4-(4-Cyanphenyl)-o-phenylen]-9α-hydroxy-4-androsten-3,17-dion, b) 11β,19-[4-(4-Cyanphenyl)-o-phenylen]-4,8-androstadien-3,17-dion, c) 11β,19-[4-(4-Cyanphenyl)-o-phenylen]-3-ethoxy-3,5,8-androstatrien-17-on und d) 11β, 19-[4-(4-Cyanphenyl)-o-phenylen]-3-ethoxy-17α-[3-(tetrahydropyran-2-yloxy)prop-1-inyl]-3,5,8-androstatrien-17β-ol [2] 854 mg der Titelverbindung als gelblicher Schaum dargestellt.

$^1$H-NMR ($CDCl_3$) [δ] ppm: 7,65-7,77 (4H, m); 7,4-7,53 (2H, m); 7,26 (1H, s); 5,93 (1H, s); 4,35 (2H, d J=5Hz); 3,81 (1H, d breit J=7,5Hz); 3,22 (1H, d J=17,5Hz); 3,05 (1H, d J=17,5Hz); 0,58 (3H, s)

**B)** Alternativ kann die Titelverbindung auch über a) Darstellung von 17β-Hydroxy-17α-(3-hydroxyprop-1-inyl)-11β, 19-[4-tri-n-butyls tannyl-o-phenylen]-4,8-androstadien-3-on [3] und b) Kupplung mit 4-Brombenzonitril analog den unter Beispiel 7 a) und b) beschriebenen Vorschriften erhalten werden. Man erhält so ausgehend von 2 g des in Beispiel 8 a) dargestellten Triflats 1,14 g der Titelverbindung.

**Beispiel 6**

**11β,19-[4-(4-Cyanphenyl)-o-phenylen]-17β-hydroxy-17α-(3-hydroxyprop-1(Z)-enyl-4,8-androstadien-3-on**

500 mg 11β,19-[4-(4-Cyanphenyl)-o-phenylen]-17β-hydroxy-17α-(3-hydrox-1-inyl)-4,8-androstadien-3-on werden in 20 ml Tetrahydrofuran gelöst, mit 0,5 ml Pyridin versetzt und unter Verwendung von 50 mg Palladium (10%-ig) auf

[2] Die Addition von 3-(Tetrahydropyran-2-yloxy)-prop-1-in erfolgt analog Vorschrift 1 d). Es wird mit einem 19-fachen Überschuß des korrespondierenden Lithiumacetylids gearbeitet.
[3] $^1$H-NMR ($CDCl_3$) [δ] ppm 7,28 (2H, s); 7,07 (1H, s); 5,9 (1H, s); 4,38 (2H d J=6Hz); 3,74 (1H, m); 3,12 (1H, d J=17,5Hz); 2,96 (1H, d J=17,5Hz); 0,87 (9H, tr J=7,5Hz); 0,54 (3H, s)

Bariumsulfat als Katalysator bei Normaldruck hydriert. Nach Aufnahme eines Äquivalents Wasserstoff wird das Reaktionsgemisch über Celite filtiert, der Filterrückstand mit Ethylacetat und Methylenchlorid nachgewaschen und das Filtrat am Vakuum eingeengt. Der Rückstand wird an Kieselgel mit einem Gemisch aus Ethylacetat/Hexan chromatographiert. Es werden 412 mg der Titelverbindung erhalten.
Fp.: 214-216 °C (Ethylacetat)
$[\alpha]^{20}_D$ = +213° ($CHCl_3$;c=0.52)

**Beispiel 7**

**<u>17β-Hydroxy-17α-(prop-1-inyl)-11β,19-[4-(5-pyrimidinyl)-o-phenylen]-4,8-androstadien-3-on</u>**

a) 17β-Hydroxy-17α-(prop-1-iny)-11β,19-(4-trifluormethylsulfonyloxy-o-phenylen)-4,8-androstadien-3-on

In analoger Sequenz der unter Beispiel 1 beschriebenen Reaktionsvorschriften werden ausgehend von 15 g 11β,19-(4-Trifluormethylsulfonyloxy-o-phenylen)-5α,9α-dihydroxy-3,3-(2,2-dimethyltrimethylendioxy)-androstan-17-on über 1) 11β,19-(4-Trifluormethylsulfonyloxy-o-phenylen)-9α-hydroxy-4-androsten-3,17-dion [4], 2) 11β,19-(4-Trifluormethylsulfonyloxy-o-phenylen)-4,8-androstadien-3,17-dion [5], 3) 3-Ethoxy-11β,19-(4-trifluormethylsulfonyloxy-o-phenylen)-3,5,8-androstatrien-17-on und 4) 3-Ethoxy-17α-(prop-1-inyl)-11β,19-(4-trifluormethylsulfonyloxy-o-phenylen)-3,5,8-androstatrien-17β-ol 3,7 g 17β-Hydroxy-17α-(prop-1-inyl)-11β,19-(4-trifluormethylsulfonyloxy-o-phenylen)-4,8-androstadien-3-on als gelblicher Schaum dargestellt.
$^1$H-NMR ($CDCl_3$) [δ] ppm: 7,44 (1H, d J=8Hz); 7,11 (1H, dd J=-8Hz und $J_1$=2Hz); 6,96 (1H d $J_1$=2Hz); 5,92 (1H, s); 3,74 (1H, m); 3,15 (1H, d J=17,5Hz); 2,99 (1H, d J=17,5Hz); 1,9 (3H, s); 0,5 (3H, s)

b)17β-Hydroxy-17α-(prop-1-inyl)-11β,19-(4-tri-n-butylstannyl-o-phenylen)-4,8-androstadien-3-on

3,5 g des unter a) dargestellten Triflats werden in 225 ml absolutem Dioxan gelöst und mit 1 g Lithiumchlorid und 480 mg Tetrakistriphenylphosphinpalladium versetzt. Nach fünfminütigem Nachrühren wird das Reaktionsgemisch mit 12 ml Hexa-n-butyldizinn versetzt, 2,5 Stunden bei Rückfluß unter Schutzgas gerührt, auf Raumtemperatur abgekühlt und mit Ethylacetat verdünnt. Nach Filtration über Celite und Waschen des Filterrückstandes mit Ethylacetat wird die organische Phase mit gesättigter Natriumchloridlösung gewaschen, über Natriumsulfat getrocknet und am Vakuum eingeengt Chromatographie des Rückstandes an Kieselgel mit einem Gemisch aus Ethylacetat/Hexan ergibt 2,86 g 17β-Hydroxy-17α-(prop-1-inyl)-11β,19-(4-tri-n-butylstannyl-o-phenylen)-4,8-androstadien-3-on als gelblichen Schaum.
$^1$H-NMR ($CDCl_3$) [δ] ppm: 7,28 (2H, s); 7,06 (1H, s); 5,89 (1H, s); 3,73 (1H, m); 3,13 (1H, d J=17,5Hz); 2,95 (1H, d J=17,5Hz); 1,9 (3H, s); 0,87 (9H, tr J=7,5Hz); 0,53 (3H, s)

c) 17β-Hydroxy-17α-(prop-1-inyl)-11β,19-[4-(5-pyrimidinyl)-o-phenylen]-4,8-androstadien-3-on

2,8 g der unter b) dargestellten ßnnverbindung werden in 100 ml absolutem Toluol gelöst, mit 6,48 g 5-Brompyrimidin und 110 mg Tetrakistriphenylphosphinpalladium versetzt und das Reactionsgemisch 14 Stunden zum Rückfluß erhitzt. Anschließend wird es auf Raumtemperatur abgekühlt, am Vakuum eingeengt und der Rückstand an Kieselgel mit einem Gemisch aus Ethylacetat/Hexan chromatographiert Es werden 1,35 g der Titelverbindung als gelblicher Schaum isoliert.
$^1$H-NMR ($CDCl_3$) [δ] ppm: 9,2 (1H, s); 8,95 (2H, s); 7,4-7,72 (3H, m); 5,93 (1H, s); 3,83 (1H, d breit J=7,5Hz); 3,22 (1H, d J=17,5Hz); 3,07 (1H, d J=17,5Hz); 1,93 (3H, s); 0,57 (3H, s)

**Beispiel 8**

**<u>17β-Hydroxy-17α-(3-hydroxyprop-1-inyl)-11β,19-[4-(3-pyrodyl)-o-phenylen]-4,8-androstadien-3-on</u>**

a)17β-Hydroxy-17α-(3-hydroxyprop-1-inyl)-11β,19-(4-trifluormethylsulfonyloxy-o-phenylen)-4,8-androstadien-3-on

Analog Beispiel 6 a) bzw. Beispiel 1 werden über Addition von 3-(Tetrahydropyran-2-yloxy)-prop-1-in (s. Beispiel 5) anstelle von Prop-1-in ausgehend von 10 g 11β,19-(4-Trifluormethylsulfonyloxy-o-phenylen)-4,8-androstadien-

**4** $^1$H-NMR ($CDCl_3$) [δ] ppm: 7,52 (1H, d J=8,5Hz); 7,12 (1H, dd J=8,5Hz und $J_1$=2Hz); 7,06 (1H, d [$J_1$=2Hz); 6,02 (1H, s); 3,5 (1H, d J=17,5Hz); 3,2 (1H, d J=5Hz); 2,96 (1H, d J=17,5Hz); 0,34 (3H, s)
**5** $^1$H-NMR ($CDCl_3$) [δ] ppm: 7,42 (1H, d J=8,5Hz); 7,13 (1H, dd J=8,5Hz und $J_1$=2Hz); 7,96 (1H, d $J_1$=2Hz); 5,95 (1H, s); 3,77 (1H, m); 3,15 (1H, d J=17,5Hz); 2,99 (1H, d J=17,5Hz); 0,58 (3H, s)

3,17-dion 5,66 g 17β-Hydroxy-17α-(3-hydroxyprop-1-inyl)-11β,19-(4-trifluormethylsulfonyloxy-o-phenylen)-4,8-androstadien-3-on als gelblicher Schaum erhalten.
$^1$H-NMR ($CDCl_3$) [δ] ppm: 7,44 (1H, d J=8,5Hz); 7,12 (1H, d J=8,5Hz und $J_1$=2Hz); 6,96 (1H, d J=2Hz); 5,91 (1H, s); 4,37 (2H, s); 3,84 (3H, s); 3,77 (1H, m); 3,16 (1H, d J=17,5Hz); 3,0 (1H, d J=17,5Hz); 0,52 (3H, s)

b) 17β-Hydroxy-17α-(3-hydroxyprop-1-inyl)-11β,19-[4-(3-pyridyl)-o-phenylen]-4,8-androstadien-3-on

1 g des unter a) dargestellten Triflats werden in einem Gemisch aus 20 ml Toluol und 10 ml Ethanol gelöst und nacheinander mit 110 mg Palladiumtetrakistriphenylphosphin, 155 mg Lithiumchlorid, 2,5 ml 2 m Natriumcarbonatlösung und 290 mg Diethyl-(3-pyridyl)-boran versetzt. Das Reaktionsgemisch wird dann 1 Stunde bei 110 °C gerührt, auf Raumtemperatur abgekühlt und mit gesättigter Natriumchloridlösung versetzt Die organische Phase wird abgetrennt, nacheinander mit 5 %-iger Natronlauge und Wasser gewaschen, über Natriumsulfat gewaschen und am Vakuum eingeengt. Der Rückstand wird an Kieselgel mit einem Gemisch aus Ethylacetat/Hexan chromatographiert. Man erhält 640 mg der Titelverbindung als gelblichen Schaum.

$^1$H-NMR ($CDCl_3$) [δ] ppm: 8,83 (1H, s breit); 8,57 (1H, dd $J_1$=2 und $J_2$=5Hz); 7,89 (1H, dtr $J_1$=2 und $J_2$=7,5Hz); 7,33-7,52 (3H, m); 7,26 (1H, s breit); 5,91 (1H, s); 4,34 (2H, d J=5Hz); 3,8 (1H, d breit J=7,5Hz); 3,21 (1H, d J=17,5Hz); 3,06 (1H, d J=17,5Hz); 0,57 (3H, s)

**Beispiel 9**

**17β-Hydroxy-17α-(3-hydroxyprop-1-inyl)-11β,19-[4-(4-methoxyphenyl)-o-phenylen]-4,8-androstadien-3-on**

Analog der unter Beispiel 8 b) beschriebenen Kupplungsvorschrift werden 1 g des unter 8 a) dargestellten Triflats mit 300 mg 4-Methoxyphenylboronsäure zu 491 mg der Titelverbindung umgesetzt

$^1$H-NMR ($CDCl_3$) [δ] ppm: 7,51 (2H, d J=8,5Hz); 7,36-7,46 (2H, m); 7,22 (1H, s breit); 6,97 (2H, d J=8,5Hz); 5,92 (1H, s); 4,39 (2H, d J=5Hz); 3,84 (3H, s); 3,79 (1H, d breit J=7,5Hz); 3,18 (1H, d J=17,5Hz); 3,04 (1H, d J=17,5Hz); 0,59 (3H, s)

**Beispiel 10**

**17β-Hydroxy-17α-(3-hydroxyprop-1-inyl)-11β,19-[4-(4-methylthiophenyl)-o-pheny-len]-4,8-androstadien-3-on**

Analog der unter Beispiel 8 b) beschriebenen Kupplungsvorschrift werden 1 g des unter 8 a) dargestellten Triflats mit 330 mg 4-Methylthiophenylboronsäure zu 567 mg der Titelverbindung umgesetzt.

$^1$H-NMR ($CDCl_3$) [δ] ppm: 7,51 (2H, d J=8,5Hz); 7,37-7,47 (2H, m); 7,3 (2H, d J=8,5Hz); 7,24 (1H, s breit); 5,92 (1H, s); 4,4 (2H, s); 3,8 (1H, d breit J=7,5Hz); 3,18 (1H, d J=17,5Hz); 3,04 (1H, d J=17,5Hz); 2,52 (3H, s); 0,57 (3H, s)

**Beispiel 11**

**11β,19-(4-Acetyl-o-phenylen)-17β-hydroxy-17α-(3-hydroxyprop-1-inyl)-4,8-androstadien-3-on**

1 g des unter Beispiel 8 a) dargestellten Triflats werden in 15 ml absolutem Dimethylformamid gelöst und mit 150 mg Lithiumchlorid und 55 mg Tetrakistriphenylphosphinpalladium versetzt Nach fünfminütigem Nachrühren wird das Reaktionsgemisch mit 0,8 ml (1-Ethoxyvinyl)-tri-n-butylzinn versetzt, 1,5 Stunden bei 110 °C unter Schutzgas gerührt, auf Raumtemperatur abgekühlt und in 1 n wäßrige Salzsäurelösung eingerührt. Die wäßrige Phase wird mehrmals mit Essigester extrahiert. Die vereinigten organischen Phasen werden mit gesättigter Natriumhydrogencarbonatlösung und gesättigter Natriumchloridlösung neutralgewaschen, über Natriumsulfat getrocknet und am Vakuum eingeengt. Chromatographie des Rückstandes an Kieselgel mit einem Gemisch aus Ethylacetat/Hexan ergibt 489 mg der Titelverbindung als gelblichen Schaum.

$^1$H-NMR ($CDCl_3$) [δ] ppm: 7,79 (1H, d J=-8,5Hz);7,65 (1H, s); 7,47 (1H, d J=8,5Hz); 5,92 (1H, s); 4,38 (2H, s); 3,8 (1H, d breit J=7,5Hz); 3,18 (1H, d J=17,5Hz); 3,05 (1H, d J=17,5Hz); 2,59 (3H, s); 0,53 (3H, s)

**Beispiel 12**

**17β-Hydroxy-17α-(3-hydroxyprop-1(Z)-enyl)-11β,19-[4-(3-pyridyl)-o-phenylen]-4,8-androstadien-3-on**

Analog der unter Beispiel 6 beschriebenen Reaktionsvorschrift werden 350 mg des unter Beispiel 8 b) erhaltenen Acetylens hydriert. Nach Chromatographie an Kieselgel mit einem Gemisch aus Essigester/Hexan werden 295 mg

der Titelverbindung als gelblicher Schaum isoliert.

$^1$H-NMR ($CDCl_3$) [δ] ppm: 8,82 (1H, s breit); 8,54 (1H, dd $J_1$=2 und $J_2$=5Hz); 7,88 (1H, dtr $J_1$=2 und $J_2$=7,5Hz); 7,32-7,51 (3H, m); 7,25 (1H, s breit); 5,9 (1H, s); 5,7-5,84 (2H, m); 4,32-4,42 (2H, m); 3,79 (1H, d breit J=7,5Hz); 3,21 (1H, d J=17,5Hz); 3,06 (1H, d J=17,5Hz); 0,65 (3H, s)

**Beispiel 13**

**17β-Hydroxy-17α-(3-hydroxyprop-1(Z)-enyl)-11β,19-[4-(4-methoxyphenyl)-o-phenylen]-4,8-androstadien-3-on**

Analog der unter Beispiel 6 beschriebenen Reaktionsvorschrift werden 250 mg des unter Beispiel 9 erhaltenen Acetylens hydriert. Nach Chromatopaphie an Kieselgel mit einem Gemisch aus Essigester/Hexan werden 175 mg der Titelverbindung als gelblicher Schaum isoliert.

$^1$H-NMR ($CDCl_3$) [δ] ppm: 7,52 (2H, d J=8,5Hz); 7,35-7,45 (2H, m); 7,22 (1H, s breit); 6,98 (2H, d J=8,5Hz); 5,91 (1H, s); 5,69-5,82 (2H, m); 4,33-4,44 (2H, m); 3,85 (3H, s); 3,78 (1H, d breit J=7,5Hz); 3,18 (1H, d J=17,5Hz); 3,04 (1H, d J=17,5Hz); 0,67 (3H, s)

**Beispiel 14**

**17β-Hydroxy-17α-(3-hydroxyprop-1(Z)-enyl)-11β,19-[4-(4-methylthiophenyl)-o-phenylen]-4,8-androstadien-3-on**

Analog der unter Beispiel 6 beschriebenen Reaktionsvorschrift werden 310 mg des unter Beispiel 10 erhaltenen Acetylens hydriert. Nach Chromatographie an Kieselgel mit einem Gemisch aus Essigester/Hexan werden 265 mg der Titelverbindung als gelblicher Schaum isoliert.

$^1$H-NMR ($CDCl_3$) [δ] ppm: 7,51 (2H, d J=8,5Hz); 7,37-7,47 (2H, m); 7,3 (2H, d J=8,5Hz); 7,24 (1H, s breit); 5,91 (1H, s); 5,7-5,85 (2H, m); 4,32-4,43 (2H, m); 3,8 (1H, d breit J=7,5Hz); 3,17 (1H, d J=17,5Hz); 3,03 (1H, d J=17,5Hz); 2,51 (3H, s); 0,68 (3H, s)

**Beispiel 15**

**11β,19-(4-Acetyl-o-phenylen)-17β-hydroxy-17-(3-hydroxyprop-1(Z)-enyl)-4,8-androstadien-3-on**

Analog der unter Beispiel 6 beschriebenen Reaktionsvorschrift werden 275 mg des unter Beispiel 11 erhaltenen Acetylens hydriert. Nach Chromatographie an Kieselgel mit einem Gemisch aus Essigester/Hexan werden 215 mg der Titelverbindung als gelblicher Schaum isoliert

$^1$H-NMR ($CDCl_3$) [δ] ppm: 7,8 (1H, d J=8,5Hz);7,66 (1H, s); 7,48 (1H, d J=8,5Hz); 5,91 (1H, s); 5,69-5,73 (2H, m); 4,32-4,42 (2H, m); 3,8 (1H, d breit J=7,5Hz); 3,18 (1H, d J=17,5Hz); 3,05 (1H, d J=17,5Hz); 2,6 (3H, s); 0,59 (3H, s)

**Beispiel 16**

**11β,19-(4-Acetyl-o-phenylen)-17β-hydroxy-17α-(prop-1-inyl)-4,8-androstadien-3-on**

a) 11β,19-(4-Nonafluorbutylsulfonyloxy-o-phenylen)-3,3-(2,2-dimethyltrimethylendioxy)-androstan-5α,9α,17β-triol

22,5 g 11β,19-(4-Hydroxy-o-phenylen)-3,3-(2,2-dimethyltrimethylendioxy)-androstan-5α, 9α,17β-triol werden bei 0 °C in 720 ml absolutem Tetrahydrofuran vorgelegt und mit 29 ml 1,6 molarer n-Butyllithiumlösung (Hexan) versetzt. Nach 30-minütigem Nachrühren werden 9,2 ml Nonafluorbutylsulfonylfluorid zugetropft Anschließend wird das Reaktionsgemisch über 1,5 Stunden langsam auf 15 °C erwärmt und dann in gesättigte Natriumhydrogencarbonatlösung gegossen. Nach 45-minütigem Nachrühren wird die wäßrige Phase mehrmals mit Ethylacetat extrahiert Die vereinigten organischen Phasen werden mit gesättigter Natriumchloridlösung gewaschen, über Natriumsulfat getrocknet und am Vakuum eingeengt Es werden 35,6 g der Titelverbindung als Rohprodukt erhalten.

Nach Chromatographie von 500 mg des Rohprodukts an Kieselgel mit einem Gemisch aus Ethylacetat/Hexan werden 384 mg 11β,19-(4-Nonafluorbutylsulfonyloxy-o-phenylen)-3,3-(2,2-dimethyltrimethylendioxy)-androstan-5α, 9α,17β-triol als weißer Schaum isoliert.

Fp. = 181-182°C (Diisopropylether)

$[\alpha]^{20}_D$ = 26° ($CHCl_3$;c=0,52)

b) 11β,19-(4-Nonafluorbutylsulfonyloxy-o-phenylen)-5α,9α-dihydroxy-3,3-(2,2-dimethyltrimethylendioxy)-androstan-17-on

Zu einem Gemisch aus 85 ml Pyridin und 250 ml Methylenchlorid werden bei 0 °C portionsweise 25,4 g Chromtrioxid zugegeben. Anschließend werden 35,1 g der unter a) dargestellten Verbindung, gelöst in 100 ml Methylenchlorid, langsam bei der gleichen Temperatur zugetropft und das Reaktionsgemisch über Nacht unter langsamer Erwärmung auf Raumtemperatur nachgerührt. Nach Beendigung des Rührens läßt man die festen Bestandteile sich absetzen, dekantiert die überstehende Phase ab und wäscht den Niederschlag mehrmals gründlich mit Methylenchlorid aus. Die vereinigten organischen Phasen werden durch Waschen mit 0,5 m Kaliumhydroxidlösung von restlichen anorganischen Bestandteilen befreit, mit Wasser neutral gewaschen, über Natriumsulfat getrocknet und am Vakuum eingeengt Durch Chromatographie des Rückstands an Aluminiumoxid (neutral, Stufe III) mit einem Gemisch aus Ethylacetat/Hexan werden 19,6 g der Titelverbindung erhalten.
Fp. = 175-176 °C (Ethylacetat)
$[\alpha]^{20}_D$ = 35° ($CHCl_3$;c=0,535)

c) 17β-Hydroxy-11β,19-(4-nonafluorbutylsulfonyloxy-o-phenylen)-17α-(prop-1-inyl)-4,8-androstadien-3-on

In analoger Sequenz der unter Beispiel 1 beschriebenen Reaktionsvorschriften werden ausgehend von 9,5 g 11β,19-(4-Nonafluorbutylsulfonyloxy-o-phenylen)-5α,9α-dihydroxy-3,3-(2,2-dimethyltrimethylendioxy)-androstan-17-on über 1) 11β,19-(4-Nonafluorbutylsulfonyloxy-o-phenylen)-9α-hydroxy-4-androsten-3,17-dion [6],2) 11β,19-(4-Nonafluorbutylsulfonyloxy-o-phenylen)-4,8-androstadien-3,17-dion [7], 3) 3-Ethoxy-11β,19-(4-nonafluorbutylsulfonyloxy-o-phenylen)-3,5,8-androstatrien-17-on und 4) 3-Ethoxy-17α-(prop-1-inyl)-11β,19-(4-nonafluorbutylsulfonyloxy-o-phenylen)-3,5,8-androstatrien-17β-ol 3,75 g 17β-Hydroxy-11β,19-(4-nonafluorbutylsulfonyloxy-o-phenylen)-17α-(prop-1-inyl)4,8-androstadien-3-on als weißer Schaum dargestellt.

$^1$H-NMR ($CDCl_3$ [δ] ppm: 7,44 (1H, d J=9Hz); 7,13 (1H, dd J=9Hz und $J_1$=2Hz); 6,97 (1H d $J_1$=2Hz); 5,93 (1H, s); 3,76 (1H, m); 3,17 (1H, d J=17,5Hz); 3,0 (1H, d J=17,5Hz); 1,9 (3H, s); 0,5 (3H, s)

d)11β,19-(4-Acetyl-o-phenylen)-17β-hydroxy-17-(prop-1-inyl)-4,8-androstadien-3-on

Analog der unter Beispiel 11 beschriebenen Reaktionsvorschrift werden 850 mg des unter c) erhaltenen Acetylens in 10 ml absolutem Dioxan gekuppelt. Nach Einengen des Reaktionsgemisches und Chromatographie des Rückstands an Kieselgel mit einem Gemisch aus Essigester/Hexan werden 264 mg der Titelverbindung als weißer Schaum isoliert.

$^1$H-NMR ($CDCl_3$) [δ] ppm: 7,8 (1H, d J=8,5Hz);7,65 (1H, s); 7,48 (1H, d J=8,5Hz); 5,93 (1H, s); 3,8 (1H, d breit J=7,5Hz); 3,18 (1H, d J=17,5Hz); 3,05 (1H, d J=17,5Hz); 2,6 (3H, s); 1,92 (3H, s); 0,5 (3H, s)

**Beispiel 17**

**<u>17β-Hydroxy-11β,19-[4-(4-methylthiophenyl)-o-phenylen]-17α-(prop-1-inyl)-4,8-androstadien-3-on</u>**

Analog der unter Beispiel 8 b) beschriebenen Reaktionsvorschrift werden 700 mg des unter Beispiel 16 c) erhaltenen Acetylens mit 185 mg 4-Methylthiophenylboronsäure gekuppelt. Nach Einengen des Reaktionsgemisches und Chromatographie des Rückstandes an Kieselgel mit einem Gemisch aus Essigester/Hexan werden 322 mg der Titelverbindung als gelblicher Schaum isoliert.

$^1$H-NMR ($CDCl_3$) [δ] ppm: 7,51 (1H, d J=8,5Hz); 7,42 (1H, s); 7,31 (1H, d J=8,5Hz); 5,92 (1H, s); 3,79 (1H, m); 3,18 (1H, d J=17,5Hz); 3,03 (1H, d J=17,5Hz); 2,51 (3H, s); 1,92 (3H, s); 0,57 (3H, s)

**Beispiel 18**

**<u>17β-Hydroxy-11β,19-[4-(3-furanyl)-o-phenylen]-17α-(prop-1-inyl)-4,8-androstadien-3-on</u>**

Analog der unter Beispiel 8 b) beschriebenen Reaktionsvorschrift werden 700 mg des unter Beispiel 16 c) erhaltenen Acetylens mit 0,5 ml (3-Furanyl)-tributylstannan gekuppelt. Nach Einengen des Reaktionsgemisches und Chromatographie des Rückstands an Kieselgel mit einem Gemisch aus Essigester/Hexan werden 395 mg der Titelverbindung als gelblicher Schaum isoliert.

[6] $^1$H-NMR ($CDCl_3$) [δ] ppm: 7,52 (1H, d J=-8,5m); 7,13 (1H, dd J=8,5Hz und $J_1$=2Hz); 7,07 (1H, d $J_1$=2Hz); 6,06 (1H, s); 3,51 (1H, d J=17,5Hz); 3,2 (1H, d J=5Hz); 2,98 (1H, d J=17,5Hz); 0,33 (3H, s)
[7] $^1$H-NMR ($CDCl_3$) [δ] ppm: 7,42 (1H, d J=8,5Hz); 7,14 (1H, dd J=8,5Hz und $J_1$=2Hz); 7,97 (1H, d $J_1$=2Hz); 5,95 (1H, s); 3,77 (1H, m); 3,15 (1H, d J=17,5Hz); 2,99 (1H, d J=17,5Hz); 0,59 (3H, s)

$^1$H-NMR ($CDDl_3$) [δ] ppm: 7,7 (1H, s); 7,48 (1H, tr J=1Hz); 7,36 (2H, m); 7,15 (1H, s); 6,69 (1H, m); 5,92 (1H, s); 3,77 (1H, m breit); 3,15 (1H, d J=17,5Hz); 3,0 (1H, d J=17,5Hz); 1,9 (3H, s); 0,57 (3H, s)

**Beispiel 19**

**17β-Hydroxy-17α-(prop-1-inyl)-11β,19-[4-(3-pyridinyl)-o-phenylen]-4,8-androstadien-3-on**

Analog der unter Beispiel 8 b) beschriebenen Reaktionsvorschrift werden 500 mg des unter Beispiel 16 c) erhaltenen Acetylens gekuppelt Nach Einengen des Reaktionsgemisches und Chromatographie des Rückstandes an Kieselgel mit einem Gemisch aus Essigester/Hexan werden 284 mg der Titelverbindung als gelblicher Schaum isoliert

$^1$H-NMR ($CDCl_3$) [δ] ppm: 8,83 (1H, s breit); 8,58 (1H, dd $J_1$=2 und $J_2$=5Hz); 7,89 (1H, dtr $J_1$=2 und $J_2$=7,5Hz); 7,3-7,7 (4H, m); 5,92 (1H, s); 3,8 (1H, d breit J=6,5Hz); 3,2 (1H, d J=16,5Hz); 3,05 (1H, dJ=16,5Hz); 1,91 (3H, s); 0,57 (3H, s)

**Beispiel 20**

**17β-Hydroxy-17α-methyl-11β,19-[4-(4-cyanphenyl)-o-phenylen]-4,8-androstadien-3-on**

a) 17β-Hydroxy-17α-methyl-11β,19-(4-hydroxy-o-phenylen)-4,8-androstadien-3-on

Unter Schutzgas werden 50 ml einer 1,6 m Methyllithiumlösung (Diethylether) vorgelegt und 9 g 3-Ethoxy-11β,19-(4-nonafluorbutylsulfonyloxy-o-phenylen)-3,5,8-androstatrien-17-on [Beispiel 16 c) 3)] gelöst in 80 ml absolutem Diethylether zugetropft Nach 10-minütigem Nachrühren wird das eiskalte Reaktionsgemisch auf gesättigte Natriumhydrogencarbonatlösung gegossen und die wässrige Phase mit Essigester extrahiert Die vereinigten organischen Phasen werden mit gesättigter Natriumchloridlösung gewaschen, über Natriumsulfat getrocknet und am Vakuum eingeengt. Der Rückstand wird einem Gemisch aus 225 ml Aceton und 5 ml 4 n wäßriger Salzsäure aufgenommen und 1 Stunde bei Raumtemperatur gerührt Anschließend wird das Reaktionsgemisch auf gesättigte Natriumhydrogencarbonatlösung gegossen und die wäßrige Phase mit Essigester extrahiert Die vereinigten organischen Phasen werden mit gesättigter Natriumchloridlösung gewaschen, über Natriumsulfat getrocknet und am Vakuum eingeengt. Der Rückstand wird an Kieselgel mit einem Gemisch aus Ethylacetat/Hexan chromatographiert. Es werden 1,65 g der Titelverbindung und 0,56 g 11β,19-(4-hydroxy-o-phenylen)-4,8-androstadien -3,17-dion[8] isoliert.

$^1$H-NMR ($CDCl_3$) [δ] ppm: 7,19 (1H, d J-=8,5Hz); 6,69 (1H, dd J=8,5 und $J_1$=2Hz); 6,49 (1H, d J=2Hz); 6,1 (1H, s); 5,93 (1H, s); 3,66 (1H, m breit); 3,09 (1H, d J=17,5Hz); 2,88 (1H, d J=17,5Hz); 1,31 (3H, s); 0,54 (3H, s)

b) 17β-Hydroxy-17α-methyl-11β,19-(4-trifluormethylsulfonyloxy-o-phenylen)-4,8-androstdien-3-on

1,5 g der unter a) erhaltenen Methylverbindung werden in 75 ml absolutem Methylenchlorid gelöst und mit 3,63 g 4-Dimethylaminopyridin versetzt. Unter Schutzgas wird die Lösung anschließend auf -78 °C abgekümt und durch langsames Zutropfen mit 1,05 ml Trifluormethansulfonsäureanhydrid gelöst in 10 ml absolutem Methylenchlorid versetzt. Nach 4-stündigem Nachrühren bei - 70 °C wird das Reaktionsgemisch vorsichtig auf gesättigte Natriumhydrogencrbonatlösung gegossen und die wäßrige Phase mit Methylenchlorid extrahiert Die vereinigten organischen Phasen werden mit gesättigter Natriumchloridlösung gewaschen, über Natriumsulfat getrocknet und am Vakuum eingeengt Der Rückstand wird an Kieselgel mit einem Gemisch aus Ethylacet/Hexan chromatographiert. Es werden 1,69 g der Titelverbindung isoliert.

$^1$H-NMR ($CDCl_3$) [δ] ppm: 7,43 (1H, d J=9Hz); 7,11 (1H, dd J=9Hz und [=2Hz); 6,95 (1H d $J_1$=2Hz); 5,92 (1H, s); 3,73 (1H, m); 3,17 (1H, d J=17,5Hz); 3,01 (1H, d J=17,5Hz); 1,32 (3H, s); 0,52 (3H, s)

c)17β-Hydroxy-17α-methyl-11β,19-[4-(4-cyanphenyl)-o-phenylen]-4,8-androstadien-3-on

800 mg des unter b) dargestellten Triflats werden analog der unter Beispiel 8 b) beschriebenen Reaktionsvorschrift mit 350 mg 4-Cyanphenylboronsäure[9] gekuppelt Nach Einengen des Reaktionsgemisches und Chromatographie des Rückstands an Kieselgel mit einem Gemisch aus Essigester/Hexan werden 552 mg der Titelverbindung als weißer Schaum isoliert.

$^1$H-NMR ($CDCl_3$) [δ] ppm: 7,65-7,77 (4H, m); 7,4-7,52 (2H, m); 7,28 (1H d $J_1$=2Hz); 5,93 (1H, s); 3,79 (1H, m); 3,21 (1H, d J=17,5Hz); 3,06 (1H, d J=17,5Hz); 1,34 (3H, s); 0,55 (3H, s)

[8] $^1$H-NMR ($CDCl_3$) [δ] ppm: 7,19 (1H, d J=8,5Hz); 6,72 (1H, dd J=8,5 und $J_1$=2Hz); 6,5 ($^1$H, d J=2Hz); 5,94 (1H, s); 5,59 (1H, s); 3,69 (1H, m breit); 3,08 (1H, d J=17,5Hz); 2,88 (1H, d J=17,5Hz); 0,6 (3H, s)

[9] S. Takahashi et al. Bull. Chem. Soc. Jpn. **62**, 3896 (1989)

**Beispiel 21**

**17α-Cyanmethyl-17β-hydroxy-11β,19-[4-(4-cyanphenyl)-o-phenylen]-4,8-androstadien-3-on**

a) 17α-Cyanmethyl-17β-hydroxy-11β,19-(4-hydroxy-o-phenylen)-4,8-androstadien-3-on

Unter Schutzgas werden 17,4 ml Diisopropylamin in 250 ml absolutem Tetrahydrofuran vorgelegt und bei -30 °C mit 77,5 ml einer 1,6 m n-Butyllithiumlösung (Hexan) versetzt. Zur vollständigen Deprotonierung wird die Lösung bei 0 °C 30 Minuten nachgerührt, bevor sie auf -70 °C heruntergekühlt wird. Nach Zutropfen von 6,52 ml Acetonitril wird 1 Stunde bei -70 °C nachgerührt. Anschließend werden 8 g (Rohprodukt) 3-Ethoxy-11β,19-(4-trifluromethylsulfonyloxy-o-phenylen)-3,5,8-androstatrien-17-on [Beispiel 7 a) 3)] gelöst in 65 ml absolutem Tetrahydrofuran langsam zugetropft. Nach 60minütigem Nachrühren wird das Reaktionsgemisch auf gesättigte Natriumhydrogencarbonatlösung gegossen und die wäßrige Phase mit Essigester extrahiert. Die vereinigten organischen Phasen werden mit gesättigter Natriumchloridlösung gewaschen, über Natriumsulfat getrocknet und am Vakuum eingeengt. Der Rückstand wird einem Gemisch aus 300 ml Aceton und 5 ml 4 n wäßriger Salzsäure aufgenommen und 1 Stunde bei Raumtemperatur gerührt Anschließend wird das Reaktionsgemisch auf gesättigte Natriumhydrogencarbonatlösung gegossen und die wäßrige Phase mit Essigester extrahiert. Die vereinigten organischen Phasen werden mit gesättigter Natriumchloridlösung gewaschen, über Natriumsulfat getrocknet und am Vakuum eingeengt. Der Rückstand wird an Kieselgel mit einem Gemisch aus Ethylacetat/Hexan chromatographiert Es werden 3,25 g der Titelverbindung isoliert.

$^1$H-NMR ($CDCl_3$) [δ] ppm: 7,19 (1H, d J=8,5Hz); 6,7 (1H, dd J=8,5 und $J_1$=2Hz); 6,5 (1H, d J=2Hz); 5,91 (1H, s); 5,59 (1H, s breit); 3,71 (1H, m bmit); 3,08 (1H, d J=17,5Hz); 2,9 (1H, d J=17,5Hz); 2,72 (1H, d J=17,5Hz); 2,57 (1H, d J=17,5Hz); 0,59 (3H, s)

b) 17α-Cyanmethyl-17β-hydroxy-11β,19-(4-trifluormethylsulfonyloxy-o-phenylen)-4,8-androstadien-3-on

3,1 g der unter a) erhaltenen Cyanmethylverbindung werden in 140 ml absolutem Methylenchlorid gelöst und mit 6,7 g 4-Dimethylaminopyridin versetzt. Unter Schutzgas wird die Lösung anschließend auf -78 °C abgekühlt und durch langsames Zutropfen mit 1,96 ml Trifluormethansulfonsäureanhydrid gelöst in 20 ml absolutem Methylenchlorid versetzt. Nach 5-stündigem Nachrühren bei - 70 °C wird das Reaktionsgemisch vorsichtig auf gesättigte Natriumhydrogencarbonatlösung gegossen und die wäßrige Phase mit Methylenchlorid extrahiert. Die vereinigten organischen Phasen werden mit gesättigter Natriumchloridlösung gewaschen, über Natriumsulfat getrocknet und am Vakuum eingeengt Der Rückstand wird an Kieselgel mit einem Gemisch aus Ethylacetat/Hexan chromatographiert. Es werden 2,75 g der Titelverbindung isoliert.

$^1$H-NMR ($CDCl_3$) [δ] ppm: 7,45 (1H, d J=9Hz); 7,12 (1H, dd J=9Hz und $J_1$=2Hz); 6,98 (1H d $J_1$=2Hz); 5,93 (1H, s); 3,78 (1H, m); 3,17 (1H, d J=17,5Hz); 3,03 (1H, d J=17,5Hz); 2,73 (1H, d J=17,5Hz); 2,58 (1H, d J=17,5Hz); 0,56 (3H, s)

c)17α-Cyanmethyl-17β-hydroxy-11β,19-[4-(4-cyanphenyl)-o-phenylen]-4,8-androstadien-3-on

1 g des unter b) dargestellten Triflats werden analog der unter Beispiel 8 b) beschriebenen Reaktionsvorschrift mit 350 mg 4-Cyanphenylboronsäure gekuppelt Nach Einengen des Reaktionsgemisches und Chromatographie des Rückstands an Kieselgel mit einem Gemisch aus Essigester/Hexan werden 552 mg der Titelverbindung als weißer Schaum isoliert.
Fp. = 212-213 °C (Ethylacetat)
$[\alpha]^{20}_D$ = 224° ($CHCl_3$;c=0,51)

**Beispiel 22**

**17α-Cyanmethyl-17β-hydroxy-11β,19-[4-(4-dimethylaminophenyl)-o-phenylen]-4,8-androstadien-3-on**

1 g des unter Beispiel 21 b) dargestellten Triflats werden analog der unter Beispiel 8 b) beschriebenen Reaktionsvorschrift mit 330 mg 4-Dimethylaminophenylboronsäure[10] gekuppelt. Nach Einengen des Reaktionsgemisches und Chromatographie des Rückstands an Kieselgel mit einem Gemisch aus Essigester/Hexan werden 460 mg der Titelverbindung als gelblicher Schaum isoliert.
Fp. = 289-290 °C (Ethylacetat)
$[\alpha]^{20}_D$ = 226° ($CHCl_3$;c=0,51)

[10] H.Staab et al. Liebigs AM. Chem. 753, 80 (1971)

## Patentansprüche

1. 8-En-19,11β-überbrückte Steroide der allgemeinen Formel I

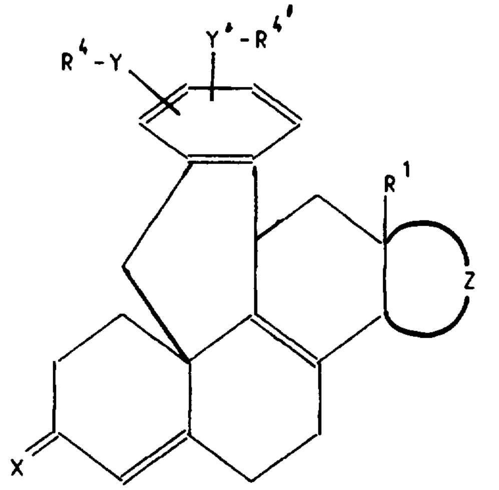

(I),

worin

$R^1$ für einen Methyl- oder Ethylrest,
X für ein Sauerstoffatom, eine Hydroxyiminogruppe oder zwei Wasserstoffatome,
Z für den Rest eines Ringes der Formel

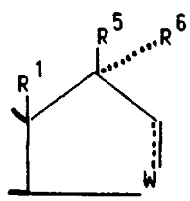

steht, worin
$R^1$ die in Formel I genannte Bedeutung hat, die von W ausgehende gestrichelte Linie die etwaige Anwesenheit einer Doppelbindung bedeutet,
W einen $CH_2$-, CH-, $CH_2CH_2$- oder $CHCH_2$-Rest,

$R^5/R^6$ $-OR^7/-C{\equiv}C-U$

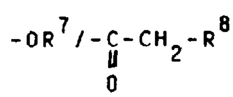

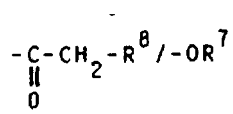

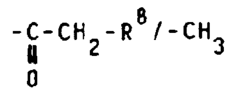

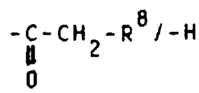

$$-OR^7/-(CH_2)_m-CH_2-R^9$$

$$-OR^7/-CH{=}CH(CH_2)_k-CH_2-R^9$$

$$-OR^{10}/-(CH_2)_k-C{\equiv}C-U$$

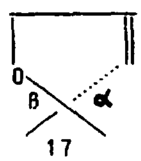 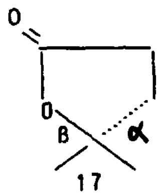 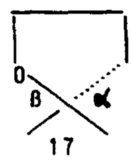

mit $R^7$ in der Bedeutung eines Wasserstoffatoms, eines Alkyl- oder Acylrestes mit 1 bis 4 Kohlenstoffatomen,
U in der Bedeutung eines Wasserstoffatoms, einer Alkyl, Hydroxyalkyl-, Alkoxyalkyl-, Acyloxyalkylgruppe mit jeweils 1 bis 4 Kohlenstoffatomen im Alkyl- bzw. Acylrest oder eines Halogenatoms,
$R^8$ in der Bedeutung eines Wasserstoffatoms, einer Hydroxygruppe, einer Alkyl-, O-Alkyl- oder O-Acylgruppe mit jeweils 1 bis 4 Kohlenstoffatomen,
$R^9$ in der Bedeutung eines Wasserstoffatoms, eines Hydroxy- oder Cyanidrestes, einer O-Alkyl- oder O-Acylgruppe mit jeweils 1 bis Kohlenstoffatomen,
$R^{10}$ in der Bedeutung eines Wasserstoffatoms, einer Alkyl- oder Acylgruppe mit jeweils 1 bis 10 Kohlenstoffatomen,
m in der Bedeutung 0, 1, 2 oder 3,
k in der Bedeutung 0, 1 oder 2,

bedeuten,
worin

$R^4$ und $R^{4'}$, die gleich oder verschieden sind, jeweils für ein Wasserstoffatom, einen Cyanidrest, eine $-OR^{11}$-, $-S(O)_kR^{11}$-, $-N(O)_nR^{11}R^{12}$-, $-O-SO_2R^{13}$-, $-P(O)(OR^{14})_2$, $-SiR^{14}_3$ oder $-SnR^{14}_3$-Gruppe steht mit n k in der Bedeutung der Ziffern 0, 1 oder 2, n in der Bedeutung der Ziffern 0 oder 1,
$R^{11}$ in der Bedeutung eines Wasserstoffatoms oder eines $C_1$-$C_8$-Alkylrestes,
$R^{12}$ in der Bedeutung von $R^{11}$, eines Cyanid- oder eines $C_1$-$C_{10}$-Acylrestes,
$R^{13}$ in der Bedeutung eines perfluorierten $C_1$-$C_4$-Alkylrestes,
$R^{14}$ in der Bedeutung eines $C_1$-$C_4$-Alkylrestes oder
$R^{11}$ und $R^{12}$ innerhalb einer $-N(O)_nR^{11}R^{12}$-Gruppe gemeinsam unter Einschluß von N einen 5- oder 6-gliedrigen heterocyclischen Ring bilden, wobei im Ring noch ein weiteres Heteroatom N, O oder S enthalten sein kann,
Y und Y', die gleich oder verschieden sind, jeweils eine direkte Bindung, eine geradkettige oder verzweigte, gegebenenfalls Doppel- oder Dreifachbindung(en) aufweisende Alkylengruppe mit bis zu 20 Kohlenstoffatomen, die gegebenenfalls substituiert ist durch eine oder mehrere Oxo-, $C_1$-$C_{10}$Acyloxy-, $-OR^{11}$-, $-S(O)_kR^{11}$- und/oder $-N(O)_nR^{11}R^{12}$-Gruppe(n), einen gegebenenfalls substituierten carbocyclischen oder heterocyclischen Arylrest oder
$R^4$-Y und $R^{4'}$-Y' gemeinsam den Rest eines, gegebenenfalls substituierten gesättigten, ungesättigten oder

aromatischen 5-oder 6-gliedrigen Ringes mit 0 bis 2 Sauerstoff-, Schwefelatomen und/oder $NR^{11}$-Gruppen bedeuten, mit der Maßgabe, daß nur dann k und n größer als 0 sind, wenn $R^{11}$ für einen $C_1$-$C_8$-Alkylrest steht,

sowie gegebenenfalls deren pharmazeutisch verträgliche Additionssalze mit Säuren.

**2.** Verbindungen nach Anspruch 1, dadurch gekennzeichnet, daß $R^2$, B und G jeweils für ein Wasserstoffatom stehen.

**3.** Verbindungen nach Anspruch 1, dadurch gekennzeichnet, daß B und G gemein sam für eine zweite Bindung und $R^2$ für ein Wasserstoffatom stehen.

**4.** Verbindungen nach Anspruch 1, dadurch gekennzeichnet, das Y und Y' je weils für eine direkte Bindung und $R^4$ und $R^{4'}$ jeweils für ein Wasserstoffatom stehen.

**5.** Verbindungen nach Anspruch 1, dadurch gekennzeichnet, daß Y und Y' je weils für eine direkte Bindung, $R^4$ für ein Wasserstoffatom und $R^{4'}$ für ein Stickstoffatom, substituiert mit zwei $C_1$-$C_8$-Alkylresten, stehen.

**6.** Verbindungen nach Anspruch 1, dadurch gekennzeichnet, daß Y und Y' je weils für eine direkte Bindung, $R^4$ für ein Wasserstoffatom und $R^{4'}$ für eine $C_1$-$C_8$-Alkoxygruppe stehen.

**7.** Verbindungen nach Anspruch 1, dadurch gekennzeichnet, daß Y für eine direkte Bindung, $R^4$ und $R^{4'}$ jeweils für ein Wasserstoffatom und Y' für eine geradkettige oder verzweigte, gegebenenfalls Doppel- und/oder Dreifachbindung(en) aufweisende Alkylengruppe mit bis zu 20 Kohlenstoffatomen, die durch eine Oxo- oder $OR^{11}$-Gruppe mit $R^{11}$ in der Bedeutung eines Wasserstoffatoms oder eines $C_1$-$C_8$-Alkylrestes, substituiert ist, stehen.

**8.** Verbindungen nach Anspruch 1, dadurch gekennzeichnet, daß $R^4$-Y und $R^{4'}$ -Y' gemeinsam für den Rest eines gesättigten, ungesättigten oder aromatischen 5- oder 6-gliedrigen Ringes mit 0 bis 2 Sauerstoff-, Schwefelatomen und/oder $NR^{11}$-Gruppen mit $R^{11}$ in der Bedeutung eines Wasserstoffatoms oder eines $C_1$-$C_8$-Alkylrestes stehen.

**9.** Verbindungen nach Anspruch 1, dadurch gekennzeichnet, daß Y'-$R^{4'}$ ein Wasserstoffatom und Y-$R^4$ eine Ethyl-, Vinyl-, Isopropyl-,Isopropenyl-, Prop1(Z)-enyl-, Prop-1(E)-enyl-, Prop-2-enyl-, Ethinyl-, Propinyl-, Prop-2-inylMethoxy-, Thiomethyl-, Thioethyl-, 1-Hydroxyethyl- oder Diethoxyphosphorylgruppe, ein substituierter oder unsubstituierter carbocyclischer oder heterocyclischer Arylrest ist.

**10.** Verbindungen nach Anspruch 9, dadurch gekennzeichnet, daß der Arylrest ein Phenyl-, Naphthyl-, 2-Methoxyphenyl-, 3-Methoxyphenyl-, 4-Methoxyphenyl-, 2-Tolyl-, 3-Tolyl-, 4-Tolyl-, 2-Dimethylaminophenyl-, 3-Dimethylaminophenyl-, 4-Dimethylaminophenyl-, Furyl-2-, Furyl-3-, Thienyl-2-, Thienyl-3-, Pyridyl-2-, Pyridyl-3-, Pyridyl-4-, Pyrimidinyl, Thiazolyl-, Imidazolyl-Rest ist.

**11.** Verbindungen nach Anspruch 1:

17β-Hydroxy-11β,19-(4-methoxy-o-phenylen)-17α-(prop-1-inyl)-4,8-androstadien-3-on
11β,19-(4-Ethyl-o-phenylen)-17β-hydroxy-17α-(prop-1-inyl)-4,8-androstadien3-on
11β,19-[4-(4-Cyanpheny)-o-phenylen]-17β-hydroxy-17α-(prop-1-inyl)-4,8-androstadien-3-on
11β,19-[4-(4-Cyanphenyl)-o-phenylen]-17α-(ethinyl)-17β-hydroxy-4,8-androstadien-3-on
11β,19-[4-(4-Cyanphenyl)-o-phenylen]-17β-hydroxy-17α-(3-hydroxyprop-1-inyl)-4,8-androstadien-3-on
11β,19-[4-(4-Cyanphenyl)-o-phenylen]-17β-hydroxy-17α-(3-hydroxyprop-1(Z)-enyl)-4,8-androstadien-3-on
17β-Hydroxy-17α-(prop-1-inyl)-11β,19-[4-(5-pyrimidynil)-o-phenylen]-4,8-androstadien-3-on
17β-Hydroxy-17α-(3-hydroxyprop-1-inyl)-11β,19-[4-(3-pyridyl)-o-phenylen]-4,8-androstadien-3-on
17β-Hydroxy-17α-(3-hydroxyprop-1-inyl)-11β,19-[4-(4-methoxyphenyl)-o-phenylen]-4,8-androstadien-3-on
17β-Hydroxy-17α-(3-hydroxyprop-1-inyl)-11β,19-[4-(4-methylthiophenyl)-o-phenylen]-4,8-androstadien-3-on
11β,19-(4-Acetyl-o-phenylen)-17β-hydroxy-17α-(3-hydroxyprop-1-inyl)-4,8-androstadien-3-on
17β-Hydroxy-17α-(3-hydroxyprop-1(Z)-enyl)-11β,19-[4-(3-pyridyl)-o-phenylen]-4,8-androstadien-3-on
17β-Hydroxy-17α-(3-hydroxyprop-1(Z)-enyl)-11β,19-[4-(4-methoxyphenyl)-o-phenylen]-4,8-androstadien-3-on
17β-Hydroxy-17α-(3-hydroxyprop-(Z)-enyl)-11β,19-[4-(4-methylthiophenyl)-o-phenylen]-4,8-androstadien-3-on
11β,19-(4-Acetyl-o-phenylen)-17β-hydroxy-17α-(3-hydroxyprop-1(Z)-enyl)-4,8-androstadien-3-on
11β,19-(4-Acetyl-o-phenylen)-17β-hydroxy-17α-(prop-1-inyl)-4,8-androstadien-3-on

17β-Hydroxy-11β,19-[4-(4-methylthiophenyl)-o-phenylen]-17α-(prop-1-inyl)-4,8-androstadien-3-on
17β-Hydroxy-11β,19-[4-(3-furanyl)-o-phenylen]-17α-(prop-1-inyl)-4,8-androstadien-3-on
17β-Hydroxy-17α-(prop-1-inyl)-11β,19-[4-(3-pyridyl)-o-phenylen]-4,8-androstadien-3-on
17β-Hydroxy-17α-methyl-11β,19-[4-(4-cyanphenyl)-o-phenylen]-4,8-androstadien-3-on
17α-Cyanmethyl-17β-hydroxy-11β,19-[4-(4-cyanphenyl)-o-phenylen]-4,8-androstadien-3-on
17α-Cyanphenyl-17β-hydroxy-11β,19-[4-(4-dimethylaminophenyl)-o-phenylen]-4,8-androstadien-3-on
17β-Hydroxy-17α-(3-hydroxyprop-1(Z)-enyl)-11β,19-(4-vinylphenyl-o-phenylen)-4,8-androstadien-3-on
17β-Hydroxy-11β,19-[4-(4-methylthiophenyl)-o-phenylen]-17α-(prop-1-inyl)-4,8-androstadien-3-on
17β-Hydroxy-17α-(3-hydroxyprop-1(Z)-enyl)-11β,19-(4-methylsulfinylphenyl-o-phenylen)-4,8-androstadien-3-on
17β-Hydroxy-17α-(3-hydroxyprop-1(Z)-enyl)-11β,19-(4-methylthio-o-phenylen)-4,78-androstadien-3-on
17β-Hydroxy-17α-(3-hydroxyprop-1(Z)-enyl)-11β,19-(4-methylsulfinyl-o-phenylen)-4,8-androstadien-3-on
11β,19-(4-Dimethylamino-o-phenylen)-17β-hydroxy-17α-(3-hydroxyprop-1(Z)-enyl)-4,8-androstadien-3-on
11β,19-[4-(4-Dimethylamminophenyl)-o-phenylen]-17β-hydroxy-17α-(3-hydroxyprop-1(Z)-enyl)-4,8-androstadien-3-on
11β,19-[4-(3-Furyl)-o-phenylen]-17β-hydroxy-17α-(3-hydroxyprop-1(Z)-enyl)-4,8-androstadien-3-on
11β,19-[4-(4-Cyanphenyl)-o-phenylen]spiro[androsta-4,8-dien-17β,2'(5'H)furan]-3-on
11β,19-[4-(4-Cyanphenyl)-o-phenylen]-4',5'-dihydrospiro[androsta-4,8-dien-17β,2'(3'H)-furan]-3-on
11β,19-[4-(4-Acetylphenyl)-o-phenylen]-17β-hydroxy-17α-(3-hydroxyprop-1-inyl)4,8-androstadien-3-on
11β,19-[4(4-Acetylphenyl)-o-phenylen]-17β-hydroxy-17α-(3-hydroxyprop-1(Z)-enyl)-4,8-androstadien-3-on
11β,19-[4-(4-Acetylphenyl)-o-phenylen]spiro[androsta-4,8-dien-17β,2'(5'H)-furan]-3-on
11β,19-[4-(4-Acetylphenyl)-o-phenylen]-4',5'-dihydrospiro[androsta-4,8-dien-17β,2'-(3'H)furan]-3-on
11β,19-[4-(4-Methylthiophenyl)-o-phenylen]spiro[androsta-4,8-dien-17β,2'(5'H)-furan]-3-on
11β,19-[4(4-Methylthiophenyl)-o-phenylen]-4',5'-dihydrospiro[androsta-4,8-dien-17β,2'-(3'H)furan]-3-on
11β,19-(4-Acetyl-o-phenylen)spiro[androsta-4,8-dien-17β,2'(5'H)-furan]-3-on
11β,19-(4-Acetyl-o-phenylen)-4',5'-dihydrospiro[androsta-4,8-dien-17β,2'(3'H)-furan]-3-on

**12.** Verbindungen der allgemeinen Formel III

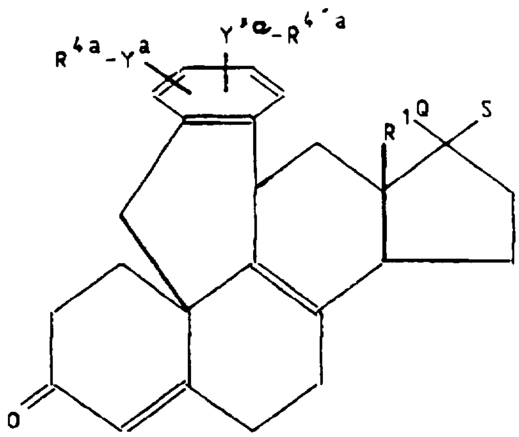

(III),

worin

$R^{4a}$, $R^{4'a}$, $Y^a$ und $Y'^a$ die gleiche Bedeutung wie $R^4$, $R^{4'}$, Y und Y' haben, wobei gegebenenfalls vorhandene Hydroxy-, Mercapto-. Amino-, Oxo- und/ oder terminale Acetylengruppen geschützt sind, oder $R^{4a}$-$Y^a$- und/ oder $R^{4'a}$-$Y'^a$ je eine Methoxy-, Hydroxy- oder Perfluoralkylsulfonatgruppe.
Q und S gemeinsam ein Sauerstoffatom oder eine der unter $R^5/R^6$ genannten Substituentenkombinationen die den nachfolgenden Reaktionsschritt der Wasserabspaltung unbeschadet übersteht oder in welchem freie Hydroxygruppen geschützt sind,

bedeuten.

**13.** Verfahren zur Herstellung von Verbindungen der allgemeinen Formel I

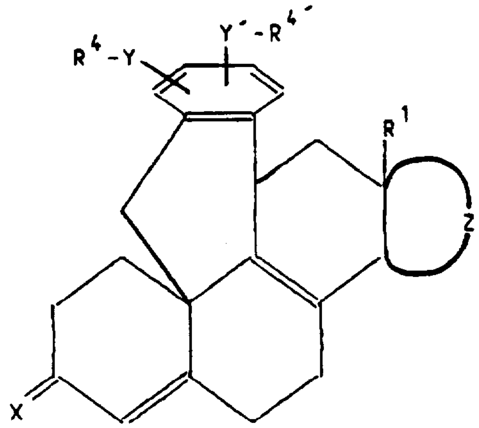

(I),

worin
$R^1$, X, Z, $R^4$ und $R^{4'}$ sowie Y und Y' die bereits in Anspruch 1 angegebene Bedeutung haben,

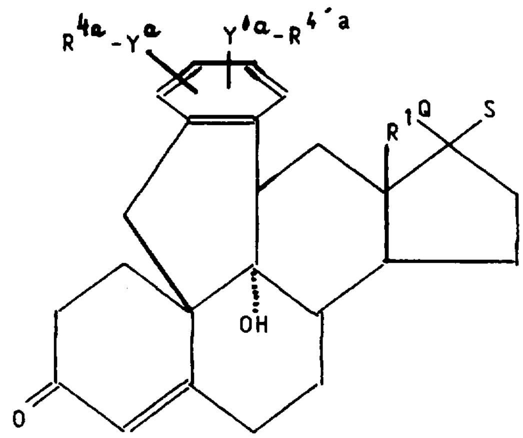

(II),

worin

$R^{4a}$, $R^{4'a}$, $Y^a$ und $Y'^a$ die gleiche Bedeutung wie $R^4$, $R^{4'}$, Y und Y' haben, wobei gegebenenfalls vorhandene Hydroxy-, Mercapto-, Amino-, Oxo- und/ oder terminale Acetylengruppen geschützt sind, oder $R^{4a}$-$y^a$- und/ oder
$R^{4'a}$-$Y'^a$ je eine Methoxy-, Hydroxy- oder Perfluoralkylsulfonatgruppe,
Q und S gemeinsam ein Sauerstoffatom oder eine der unter $R^5$/$R^6$ genannten Substituentenkombinationen die den nachfolgenden Reaktionsschritt der Wasserabspaltung unbeschadet übersteht oder in welchem freie Hydroxygruppen geschützt sind,

bedeuten,
durch Umsetzung mit einem wasserentziehenden Mittel in die entsprechende 8-En-Verbindung der allgemeinen Formel III

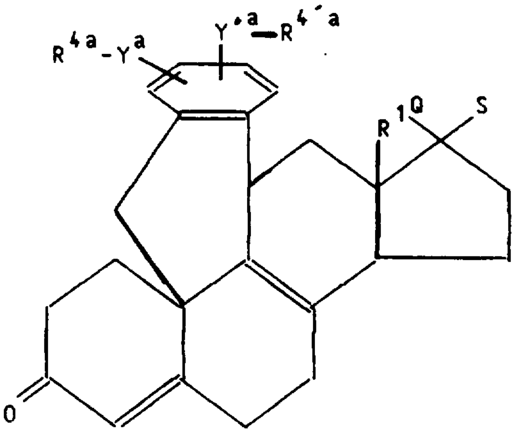

(III),

überführt wird
und anschließend gegebenenfalls

wenn Q und S gemeinsam ein Sauerstoffatom bedeuten, die 3-Ketofunktion selektiv in Form eines entsprechenden Dienolethers, Ketals oder Dithioketals geschützt und die Substituenten $R^5/R^6$ oder deren Vorläufer durch nucleophile Addition an die 17-Ketofunktion in an sich bekannter Weise (Seitenkettenaufbau) eingeführt werden, die 3-Ketogruppe wieder freigesetzt, danach gegebenenfalls, wenn $R^6$ $-(CH_2)_m-CH_2-R^9$ oder $-CH{=}CH-(CH_2)_{k-}$ $CH_2-R^9$ bedeuten soll, die entsprechende Alkinylverbindung katalytisch hydriert sowie gegebenenfalls

wenn $R^{4a}-Y^a-$ und/oder $R^{4'a}-Y'^a-$ eine Perfluoralkylsulfonatgruppe bedeutet, die gegebenenfalls zuvor aus einer Methoxy- über eine Hydroxygruppe generiert wurde, die Perfluoralkylsulfonatverbindung entweder direkt oder durch Austausch der Perfluoralkylsulfonatabgangsgruppe gegen eine Zinntrialkylgruppe über die entsprechende Zinntrialkylverbindung in eine Verbindung überführt wird, die im 19,11β-Phenylring, gegebenenfalls nach weiteren Reaktionen, das gewünschte Substitutionsmuster aufweist, wobei dieser zuletzt genannte Verfahrensschritt der Arylkupplung prinzipiell auf jeder beliebigen Stufe des erfindungsgemäßen Verfahrens durchgeführt werden kann,

das so erhaltene Produkt gegebenenfalls von Schutzgruppen befreit wird, gewünschtenfalls die im 19,11β-Phenylenring gegebenenfalls enthaltene (n) Hydroxy-, Mercapto und/oder Aminogruppe(n) alkyliert bzw. acyliert, gewünschtenfalls die in dem(n) Arylsubstituenten gegebenenfalls enthaltene(n) Amino- und/oder Sulfidgruppe(n) oxidiert werden, gewünschtenfalls mit Hydroxylamin-hydrochlorid zum Produkt der allgemeinen Formel I mit X in der Bedeutung der Hydroxyiminogruppierung >N~OH umgesetzt sowie gegebenenfalls ein pharmazeutisch verträgliches Säureadditionssalz hergestellt wird.

**14.** Verfahren nach Anspruch 13, dadurch gekennzeichnet, daß Thionylchlorid als wasserentziehendes Mittel verwendet wird.

**15.** Pharmazeutische Präparate, die mindestens eine Verbindung gemäß den Ansprüchen 1 bis 11 sowie einen pharmazeutisch verträglichen Träger enthalten.

**16.** Verwendung von Verbindungen gemäß den Ansprüchen 1 bis 11 zur Herstellung von Arzneimitteln.

## Claims

**1.** 8-Ene-19,11β-bridged steroids of the general formula I

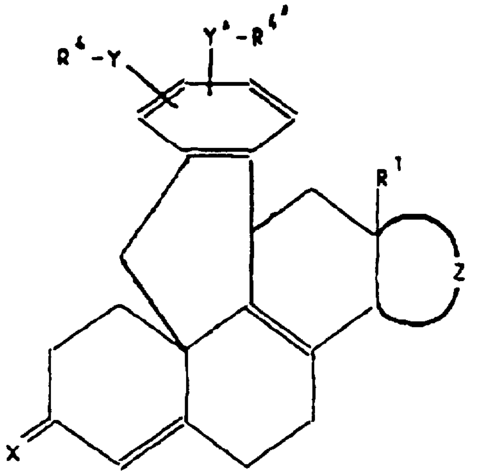

(I),

wherein

$R^1$ represents a methyl or ethyl radical,
X represents an oxygen atom, a hydroxyimino group or two hydrogen atoms,
Z represents the radical of a ring of the formula

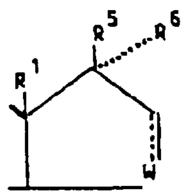

wherein
$R^1$ has the meaning given in formula I,

the dotted line starting from W indicates the possible presence of a double bond,

W represents a $CH_2$, CH, $CH_2CH_2$ or $CHCH_2$ radical,
$R^5/R^6$ represents $OR^7/-C{\equiv}C-U$

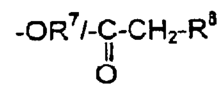

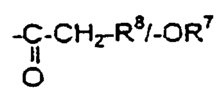

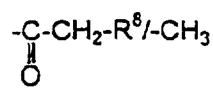

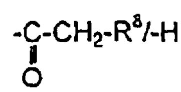

$-OR^7/-(CH_2)_m-CH_2-R^9$

$-OR^7/-CH=CH(CH_2)_k-CH_2-R^9$

$-OR^{10}/-(CH_2)_k-C{=}C-U$

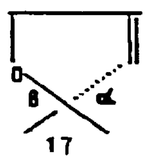

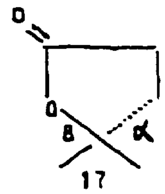

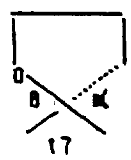

wherein

$R^7$ is a hydrogen atom, or an alkyl or acyl radical having from 1 to 4 carbon atoms,
U is a hydrogen atom, or an alkyl, hydroxyalkyl, alkoxyalkyl or acyloxyalkyl group, each of which has from 1 to 4 carbon atoms in the alkyl or acyl radical, or a halogen atom,
$R^8$ is a hydrogen atom, a hydroxy group, or an alkyl, O-alkyl or O-acyl group each of which has from 1 to 4 carbon atoms,
$R^9$ is a hydrogen atom, a hydroxy or cyanide radical, or an O-alkyl or O-acyl group each of which has from 1 to 4 carbon atoms,
$R^{10}$ is a hydrogen atom, or an alkyl or acyl group each of which has from 1 to 10 carbon atoms,
m is 0, 1, 2 or 3,
k is 0, 1 or 2,

and wherein

$R^4$ and $R^{4'}$, which are identical or different, each represent a hydrogen atom, a cyanide radical, an $-OR^{11}$, $-S(O)_kR^{11}$, $-N(O)_nR^{11}R^{12}$, $-O-SO_2R_{13}$, $-P(O)(OR^{14})_2$, $-SiR_3{}^{14}$ or $-SnR_3{}^{14}$ group wherein k is the number 0, 1 or 2 and n is the number 0 or 1,
$R^{11}$ is a hydrogen atom or a $C_1$-$C_8$alkyl radical,
$R^{12}$ has the meaning of $R^{11}$, a cyanide or a $C_1$-$C_{10}$acyl radical,
$R^{13}$ is a perfluorinated $C_1$-$C_4$alkyl radical,
$R^{14}$ is a $C_1$-$C_4$alkyl radical or
$R^{11}$ and $R^{12}$ within an $-N(O)_nR^{11}R^{12}$ group form together, and with the inclusion of N, a 5- or 6-membered heterocyclic ring, it being possible for the ring to contain a further hetero atom N, O or S,
Y and Y', which are identical or different, each represent a direct bond, a straight-chained or branched alkylene group having up to 20 carbon atoms and optionally having double or triple bond(s) that is unsubstituted or substituted by one or more oxo, $C_1$-$C_{10}$acyloxy, $-OR^{11}$, $-S(O)_kR^{11}$ and /or $-N(O)_nR^{11}R^{12}$ group(s), or an unsubstituted or substituted carbocyclic or heterocyclic aryl radical, or
$R^4$-Y and $R^{4'}$-Y' together represent the radical of an unsubstituted or substituted, saturated or unsaturated or aromatic 5- or 6-membered ring having from 0 to 2 oxygen and/or sulphur atoms and/or $NR^{11}$ groups,

with the proviso that k and n are greater than 0 only when $R^{11}$ is a $C_1$-$C_8$alkyl radical, and, where appropriate, the pharmaceutically acceptable addition salts thereof with acids.

**2.** Compounds according to claim 1, characterised in that $R^2$, B and G each represent a hydrogen atom.

**3.** Compounds according to claim 1, characterised in that B and G together represent a second bond and $R^2$ represents a hydrogen atom.

**4.** Compounds according to claim 1, characterised in that Y and Y each represent a direct bond and $R^4$ and $R^{4'}$ each represent a hydrogen atom.

**5.** Compounds according to claim 1, characterised in that Y and Y' each represent a direct bond, $R^4$ is a hydrogen atom and $R^{4'}$ represents a nitrogen atom substituted by two $C_1$-$C_8$alkyl radicals.

**6.** Compounds according to claim 1, characterised in that Y and Y' each represent a direct bond, $R^4$ represents a hydrogen atom and $R^{4'}$ represents a $C_1$-$C_8$alkoxy group.

**7.** Compounds according to claim 1, characterised in that Y represents a direct bond, $R^4$ and $R^{4'}$ each represent a hydrogen atom and Y' represents a straight-chained or branched alkylene group having up to 20 carbon atoms and optionally having double and/or triple bond(s) that is substituted by an oxo or $OR^{11}$ group wherein $R^{11}$ is a hydrogen atom or a $C_1$-$C_8$alkyl radical.

**8.** Compounds according to claim 1, characterised in that $R^4$-Y and $R^{4'}$-Y' together represent the radical of a saturated or unsaturated or aromatic 5- or 6-membered ring having from 0 to 2 oxygen and/or sulphur atoms andlor $NR^{11}$ groups wherein $R^{11}$ is a hydrogen atom or a $C_1$-$C_8$alkyl radical.

**9.** Compounds according to claim 1, characterised in that Y'-$R^{4'}$ is a hydrogen atom and Y-$R^4$ is an ethyl, vinyl, isopropyl, isopropenyl, prop-1(Z)enyl, prop-1(E)-enyl, prop-2-enyl, ethynyl, propynyl, prop-2-ynylmethoxy, thiomethyl, thioethyl, 1-hydroxyethyl or diethoxyphosphoryl group, or a substituted or unsubstituted carbocyclic or heterocyclic aryl radical.

**10.** Compounds according to claim 9, characterised In that the aryl radical is a phenyl, naphthyl, 2-methoxyphenyl, 3-methoxyphenyl, 4-methoxyphenyl, 2-tolyl, 3-tolyl, 4-tolyl, 2-dimethylaminophenyl, 3-dimethylaminophenyl, 4-dimethylaminophenyl, 2-furyl, 3-furyl, 2-thienyl, 3-thienyl, 2-pyridyl, 3-pyridyl, 4-pyridyl, pyrimidinyl, thiazolyl or imidazolyl radical.

**11.** Compounds according to claim 1:

17β-hydroxy-11β,19-(4-methoxy-o-phenylene)-17α-(prop-1-ynyl)-4,8-androstadien-3-one

11β,19-(4-ethyl-o-phenylene)-17β-hydroxy-17α-(prop-1-ynyl)-4,8-androstadien-3-one

11β,19-[4-(4-cyanophenyl)-o-phenylene]-17β-hydroxy-17α-(prop-1-ynyl)-4,8-androstadien-3-one

11β,19-[4-(4-cyanophenyl)-o-phenylene]-17α-(ethynyl)-17β-hydroxy-4,8-androstadien-3-one

11β,19-[4-(4-cyanophenyl)-o-phenylene]-17β-hydroxy-17α-(3-hydroxyprop-1-ynyl)-4,8-androstadien-3-one

11β,19-[4-(4-cyanophenyl)-o-phenylene]-17β-hydroxy-17α-(3-hydroxyprop-1(Z)-enyl)-4,8-androstadien-3-one

17β-hydroxy-17α-(prop-1-ynyl)-11β,19-[4-(5-pydmidinyl)-o-phenylene]-4,8-androstadien-3-one

17β-hydroxy-17α-(3-hydroxyprop-1-ynyl)-11β,19-[4-(3-pyridyl)-o-phenylene]-4,8-androstadien-3-one

17β-hydroxy-17α-(3-hydroxyprop-1-ynyl)-11β,19-[4-(4-methoxyphenyl)-o-phenylene]-4,8-androstadien-3-one

17β-hydroxy-17α-(3-hydroxyprop-1-ynyl)-11β,19-[4-(4-methylthiophenyl)-o-phenylene]-4,8-androstadien-3-one

11β,19-(4-acetyl-o-phenylene)-17β-hydroxy-17α-(3-hydroxyprop-1-ynyl)-4,8-androstadien-3-one

17β-hydroxy-17α-(3-hydroxyprop-1(Z)-enyl)-11β,19-[4-(3-pyridyl)-o-phenylene]-4,8-androstadien-3-one

17β-hydroxy-17α-(3-hdyroxyprop-1(Z)-enyl)-11β,19-[4-(4-methoxyphenyl)-o-phenylene]-4,8-androstadien-3-one

17β-hydroxy-17α-(3-hydroxyprop-(Z)-enyl)-11β,19-[4-(4-methylthiophenyl)-o-phenylene]-4,8-androstadien-3-one

11β,19-(4-acetyl-o-phenylene)-17β-hydroxy-17α-(3-hydroxyprop-1(Z)-enyl)4,8-androstadien-3-one

11β,19-(4-acetyl-o-phenylene)-17β-hydroxy-17α-(prop-1-ynyl)-4,8-androstadien-3-one

17β-hydroxy-11β,19-[4-(4-methylthiophenyl)-o-phenylene]-17α-(prop-1-ynyl)-4,8-androstadien-3-one

17β-hydroxy-11β,19-[4-(3-furanyl)-o-phenylene]-17α-(prop-1-ynyl)-4,8-androstadien-3-one

17β-hydroxy-17α-(prop-1-ynyl)-11β,19-[4-(3-pyridyl)-o-phenylene]-4,8-androstadien-3-one

17β-hydroxy-17α-methyl-11β,19-[4-(4-cyanophenyl)-o-phenylene]-4,8-androstadien-3-one

17α-cyanomethyl-17β-hydroxy-11β,19-[4-(4-cyanophenyl)-o-phenylene]-4,8-androstadien-3-one

17α-cyanomethyl-17β-hydroxy-11β,19-[4-(4-dimethylaminophenyl)-o-phenylene]-4,8-androstadien-3-one

17β-hydroxy-17α-(3-hydroxyprop-1(Z)-enyl)-11β,19-(4-vinylphenyl-o-phenylene)-4,8-androstadien-3-one

17β-hydroxy-11β,19-[4-(4-methylthiophenyl)-o-phenylene]-17α-(prop-1-ynyl)-4,8-androstadien-3-one

17β-hydroxy-17α-(3-hydroxyprop-1(Z)-enyl)-11β,19-(4-methylsulphinylphenyl-o-phenylene)-4,8-androstadien-3-one

17β-hydroxy-17α-(3-hydroxyprop-1(Z)-enyl)-11β,19-(4-methylthio-o-phenylene)-4,8-androstadien-3-one

17β-hydroxy-17α-(3-hydroxyprop-1(Z)-enyl)-11β,19-(4-methylsulphinyl-o-phenylene)-4,8-androstadien-3-one

11β,19-(4-dimethylamino-o-phenylene)-17β-hydroxy-17α-(3-hydroxyprop-1(Z)-enyl)-4,8-androstadien-3-one

11β,19-[4-(4-dimethylaminophenyl)-o-phenylene]-17β-hydroxy-17α-(3-hydroxyprop-1(Z)-enyl)-4,8-androstadien-3-one

11β,19-[4-(3-furyl)-o-phenylene]-17β-hydroxy-17α-(3-hydroxyprop-1(Z)-enyl)-4,8-androstadien-3-one

11β,19-[4-(4-cyanophenyl)-o-phenylene]spiro(androsta-4,8-dien-17β,2'(5'H)-furan]-3-one

11β,19-[4-(4-cyanophenyl)-o-phenylene]-4',5'-dihydrospiro[androsta-4,8-dien-17β,2'(3'H)-furan]-3-one

11β,19-[4-(4-acetylphenyl)-o-phenylene]-17β-hydroxy-17α-(3-hydroxyprop-1-ynyl)-4,8-androstadien-3-one

11β,19-[4-(4-acetylphenyl)-o-phenylene]-17β-hydroxy-17α-(3-hydroxyprop-1(Z)-enyl)-4,8-androstadien-3-one

11β,19-[4-(4-acetylphenyl)-o-phenylene]spiro[androsta-4,8-dien-17β,2'(5'H)-furan]-3-one

11β,19-[4-(4-acetylphenyl)-o-phenylene]-4',5'-dihydrospiro[androsta-4,8-diene-17β,2'(3'H)-furan]-3-one

11β,19-[4-(4-methylthiophenyl)-o-phenylene]spiro[androsta-4,8-dien-17β,2'(5'H)-furan]-3-one

11β,19-[4-(4-methylthiophenyl)-o-phenylene]-4',5'-dihydrospiro[androsta-4,8-dien-17β,2'-(3'H)-furan]-3-one

11β,19-(4-acetyl-o-phenylene)spire[androsta-4,8-dien-17β,2'(5'H)-furan]-3-one

11β,19-(4-acetyl-o-phenylene)-4',5'-dihydrospiro[androsta-4,8-dien-17β,2'(3'H)-furan]-3-one

**12.** Compounds of the general formula III

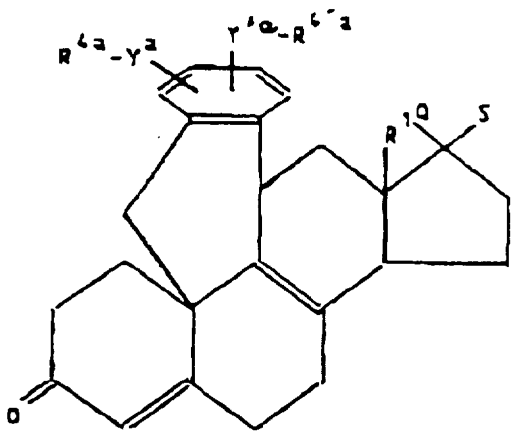

(III).

wherein

$R^{4a}$, $R^{4'a}$, $Y^a$ and $Y'^a$ have the same meaning as $R^4$, $R^{4'}$, Y and Y', any hydroxy, mercapto, amino, oxo and/or terminal acetylene groups that may be present being protected, or $R^{4a}$-$Y^a$ and/or $R^{4'a}$-$Y'^a$ each represent a methoxy-, hydroxy- or perfluoroalkyl-sulphonate group,

Q and S together represent an oxygen atom or one of the combinations of substituents mentioned under $R^5$/$R^6$ that survives undamaged the subsequent reaction step of the removal of water or in which free hydroxy groups are protected.

**13.** A process for the preparation of compounds of the general formula I

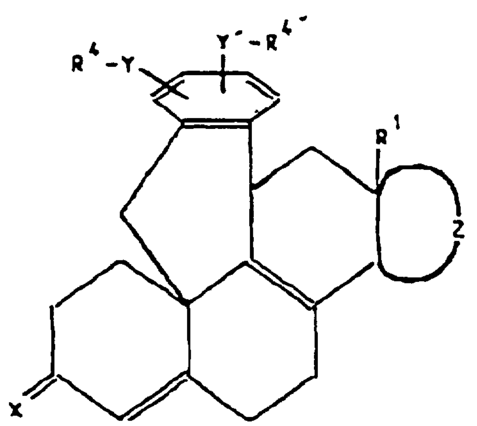

(I).

wherein

$R^1$, X, Z, $R^4$ and $R^{4'}$ and Y and Y have the meaning already indicated in claim 1, which process is characterised in that a compound of the general formula II

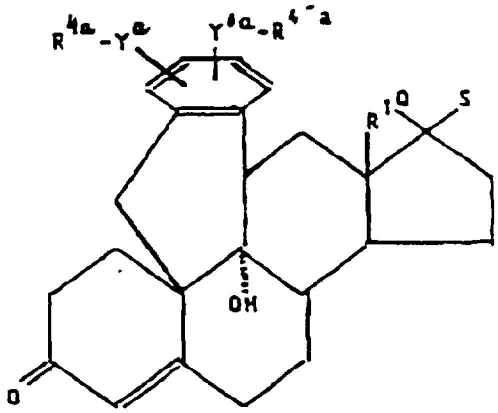

(II).

wherein

$R^{4a}$, $R^{4'a}$, $Y^a$ and $Y'^a$ have the same meaning as $R^4$, $R^{4'}$, Y and Y', any hydroxy, mercapto, amino, oxo and/or terminal acetylene groups that may be present being protected, or $R^{4a}$-$Y^a$ and/or $R^{4'a}$-$Y'^a$ each represent a methoxy-, hydroxy- or perfluoroalkyl-sulphonate group.

Q and S together represent an oxygen atom or one of the combinations of substituents mentioned under $R^5$/$R^6$ that survives undamaged the subsequent reaction step of the removal of water or in which free hydroxy groups are protected,

is converted by reaction with a water-removing agent into the corresponding 8-ene compound of the general formula III

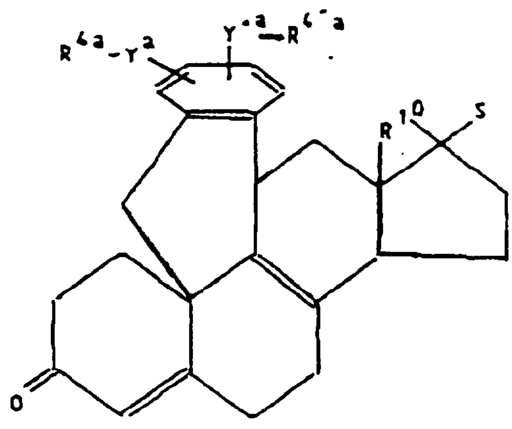

(III),

and then, optionally,

when Q and S together represent an oxygen atom, the 3-keto function is selectively protected in the form of a corresponding dienol ether, ketal or dithioketal and the substituents $R^5$/$R^6$ or the precursors thereof are introduced by nucleophilic addition to the 17-keto function in a manner known *per se* (side-chain synthesis), the 3-keto group is freed again and then, optionally, when $R^6$ is to represent $-(CH_2)_m-CH_2-R^9$ or $-CH=CH=(CH_2)_k-Ch_2-R^9$, the corresponding alkynyl compound is catalytically hydrogenated and, optionally, when $R^{4a}$-$Y^a$ and/or $R^{4'a}$-$Y'^a$ represent(s) a perfluoroalkylsulphonate group, which may previously have been generated from a methoxy group via a hydroxy group, the perfluoroalkylsulphonate compound is converted, either directly or by exchanging the perfluoroalkylsulphonate leaving group for a tin-trialkyl group via the corresponding tin-trialkyl compound, into a compound that, if necessary after further reactions, has the desired substitution pattern in the 19,11β-phenyl ring, it being possible for the last-mentioned process step of aryl coupling to be carried out in principle at any desired stage of the process according to the invention,

the product thus obtained is optionally freed of protecting groups, if desired the hydroxy, mercapto and/or

amino group(s) that may be present in the 19,11β-phenyl ring is(are) alkylated and/or acylated, if desired the amino and/or sulphide group(s) that may be present in the aryl substituent(s) are oxidised, if desired the product is reacted with hydroxylamine hydrochloride to form the product of the general formula I wherein X represents the hydroxyimino grouping >N~OH and, optionally, a pharmaceutically acceptable acid addition salt is prepared.

**14.** A process according to claim 13, characterised in that thionyl chloride is used as the water-removing agent.

**15.** Pharmaceutical preparations containing at least one compound according to claims 1 to 11 and a pharmaceutically acceptable carrier.

**16.** Use of compounds according to claims 1 to 11 in the preparation of medicaments.

**Revendications**

**1.** Stéroïdes pontés en 8-ène-19,11β de formule générale I

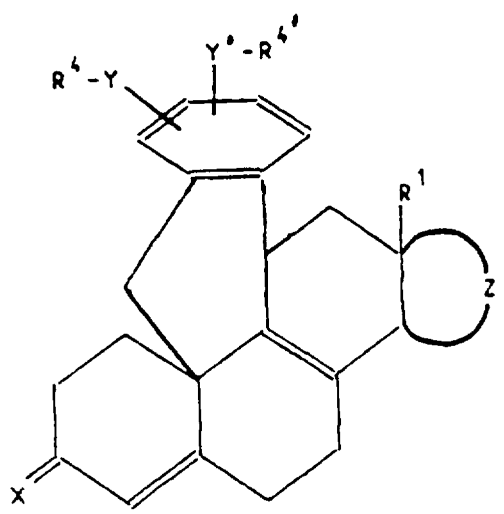

(I),

dans laquelle

$R^1$ représente un radicale méthyle ou éthyle
X représente un atome d'oxygène, un groupe hydroxylimino ou deux atomes d'hydrogène
Z représente un radical d'un cycle de formule

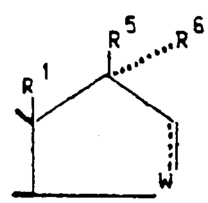

dans laquelle
$R^1$ a la signification donnée dans la formule I

la ligne en tirets partant de W signifie une présence probable de la double liaison.

W représente un radical $CH_2$-, CH-, $CH_2CH_2$- ou $CHCH_2$-, $R^5$/$R^6$ représente -$OR^7$/-C≡C-U

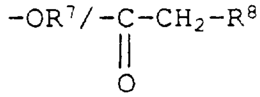

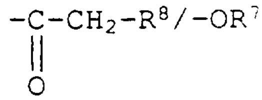

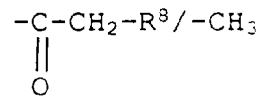

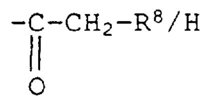

$-OR^7/-(CH_2)_m-CH_2-R^9$

$-OR^7/-CH{=}CH(CH_2)_k-CH_2-R^9$

$-OR^{10}/-(CH_2)_k-C{\equiv}C-U$

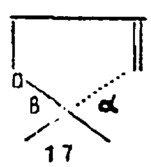

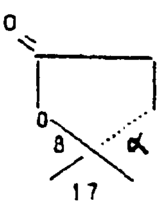

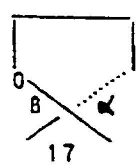

où

$R^7$ représente un atome d'hydrogène, un radical alkyle ou acyle avec 1 à 4 atomes de carbone, U représente un atome d'hydrogène, un groupe alkyle, hydroxyalkyle, alcoxyalkyle, acyloxyalkyle chacun avec 1 à 4 atomes de carbone dans le radical alkyle, respectivement acyle, ou un atome halogène,
$R^8$ représente un atome d'hydrogène, un groupe hydroxy, un groupe alkyle, O-alkyle ou O-acyle chacun avec 1 à 4 atomes de carbone,
$R^9$ représente un atome d'hydrogène, un radical hydroxy ou cyanure, groupe O-alkyle ou O-acyle chacun avec 1 à 4 atomes de carbone,
$R^{10}$ représente un atome d'hydrogène, un groupe alkyle ou acyle chacun avec 1 à 10 atomes de carbone,
m vaut 0, 1, 2 ou 3,
k vaut 0, 1 ou 2,

où

$R^4$ et $R^5$, qui sont identiques ou différents, représentent chacun un atome d'hydrogène, un radical cyanure, un groupe $-OR^{11}$, $-S(O)_kR^{11}$-, $-N(O)_nR^{11}R^{12}$-, $-O-SO_2R^{13}$-, $-P(O)(OR^{14})_2$, $-SiR_3^{14}$ ou $SnR_3^{14}$ avec k valant 0,

1 ou 2, n valant 0 ou 1,
$R^{11}$ représente un atome d'hydrogène ou un radical alkyle en $C_1$-$C_8$
$R^{12}$ a la signification de $R^{11}$, représente un radical cyanure ou un radical acyle en $C_1$-$C_{10}$,
$R^{13}$ représente un radical alkyle en $C_1$-$C_4$ perfluoré ou
$R^{14}$ représente un radical alkyle en $C_1$-$C_4$ ou
$R^{11}$ et $R^{12}$ dans un groupe -$N(O)_nR^{11}R^{12}$, ensemble avec N, forment un cycle hétérocyclique à 5 ou 6 chaînons, un autre hétéroatome N, O ou S pouvant être contenu dans le cycle,
Y et Y' qui i sont identiques ou différents, représentent chacun une liaison directe, un groupe alkylène linéaire ou ramifié comportant jusqu'à 20 atomes de carbone, présentant éventuellement une (des) double(s) ou triple (s) liaison(s), lequel groupe est éventuellement substitué par un ou plusieurs groupes oxo, acyloxy en $C_1$-$C_{10}$, -$OR^{11}$, -$S(O)_kR^{11}$- et/ou -$N(O)_nR^{11}R^{12}$, un radical acyle carbocyclique ou hétérocyclique éventuellement substitué ou
$R^4$-Y et $R^{4'}$-Y' ensemble représentent un radical d'un cycle à 5 ou 6 chaînons éventuellement substitué, saturé, insaturé ou aromatique avec 0 à 2 atomes d'oxygène, atomes de soufre et/ou groupes $NR^{11}$, à la condition que k et n ne soient supérieurs à 0 que lorsque $R^{11}$ représente un radical alkyle en $C_1$-$C_8$,

ainsi qu'éventuellement leurs sels d'addition d'acide pharmaceutiquement compatibles.

**2.** Composés selon la revendication 1, caractérisés en ce que $R^2$, B et G représentent chacun un atome d'hydrogène.

**3.** Composés selon la revendication 1, caractérisés en ce que B et G, ensemble, représentent une deuxième liaison et $R^2$ un atome d'hydrogène.

**4.** Composés selon la revendication 1, caractérisés en ce que Y et Y' chacun représentent une liaison directe et $R^4$ et $R^{4'}$ chacun un atome d'hydrogène.

**5.** Composés selon la revendication 1, caractérisés en ce que Y et Y' chacun représentent une liaison directe, $R^4$ un atome d'hydrogène et $R^{4'}$ un atome d'azote, substitué par deux radicaux alkyle en $C_1$-$C_8$.

**6.** Composés selon la revendication 1, caractérisés en ce que Y et Y' chacun représentent une liaison directe, $R^4$ un atome d'hydrogène et $R^{4'}$ un groupe alcoxy en $C_1$-$C_8$.

**7.** Composés selon la revendication 1, caractérisés en ce que Y représente une liaison directe, $R^4$ et $R^{4'}$ chacun un atome d'hydrogène et Y' représente un groupe alkylène linéaire ou ramifié avec jusqu'à 20 atomes de carbone, présentant éventuellement une (des) double(s) et/ou triple(s) liaison (s), lequel groupe est substitué par un groupe oxo ou $OR^{11}$ où $R^{11}$ étant un atome d'hydrogène ou un radical alkyle en $C_1$-$C_8$.

**8.** Composés selon la revendication 1, caractérisés en ce que $R^4$-Y et $R^{4'}$-Y' ensemble représentent le radical d'un cycle à 5 ou 6 chaînons aromatique, saturé ou insaturé, comportant 0 à 2 atomes d'oxygène, de soufre et/ou groupes $NR^{11}$ où $R^{11}$ étant un atome d'hydrogène ou un radical alkyle en $C_1$-$C_8$.

**9.** Composés selon la revendication 1, caractérisés en ce que Y'-$R^{4'}$ représente un atome d'hydrogène et -Y-$R^4$ un groupe éthyle, vinyle, isopropyle, ispropényle, prop-1(Z)-ényle, prop-1(E)-ényle, prop-2-ényle, éthynyle, propynyle, prop-2-ynyle, méthoxy, thiométhyle, thioéthyle, 1-hydroxyéthyle ou diéthoxyphosphoryle, un radical aryle carbocyclique ou hétérocyclique substitué ou non substitué.

**10.** Composés selon la revendication 9, caractérisés en ce que le radical aryle est un radical phényle, naphtyle, 2-méthoxyphényle, 3-méthoxyphényle, 4-méthoxyphényle, 2-tolyle, 3-tolyle, 4-tolyle, 2-diméthylaminophényle, 3-diméthylaminophényle, 4-diméthylaminophényle, furyle-2, furyle-3, thiényle-2, thiényle-3, pyridyle-2, pyridine-3, pyridyle-4, pyrimidinyle, thiazolyle, imidazolyle.

**11.** Composés selon la revendication 1 :

17β-hydroxy-11β,19-(4-méthoxy-o-phénylène)-17α-(prop-1-inyle)-4,8-androstadiène-3-one
11β,19-(4-éthyl-o-phénylène)-17β-hydroxy-17α-(prop-1-inyle)-4,8-androstadiène-3-one
11β,19-[4-(4-cyanophényl)-o-phénylène)-17β-hydroxy-17α-(prop-1-inyle)-4,8-androstadiène-3-one
11β,19-[4-(4-cyanophényl)-o-phénylène)-17α-(éthynyle)-17β-hydroxy-4,8-androstadiène-3-one
11β,19-[4-(4-cyanophényl)-o-phénylène)-17β-hydroxy-17α-(3-hydroxyprop-1-inyl)-4,8-androstadiène-3-one

11β,19-[4-(4-cyanophényl)-o-phénylène)-17β-hydroxy-17α-(3-hydroxyprop-1(Z)-ényl)-4,8-androstadiène-3-one
17β-hydroxy-17α-(prop-1-inyl)-11β,19-[4-(5-pyrimidinyl)-o-phénylène]-4,8-androstadiène-3-one
17β-hydroxy-17α-(3-hydroxyprop-1-inyl)-11β,19-[4-(3-pyridyl)-o-phénylène]-4,8-androstadiène-3-one
17β-hydroxy-17α-(3-hydroxyprop-1-inyl)-11β,19-[4-(4-méthoxyphényl)-o-phénylène]-4,8-androstadiène-3-one
17β-hydroxy-17α-(3-hydroxyprop-1-inyl)-11β,19-[4-(4-méthylthiophényl)-o-phénylène]-4,8-androstadiène-3-one
11β,19-(4-acétyl-o-phénylène)-17β-hydroxy-17α-(3-hydroxyprop-1-inyl)-4,8-androstadiène-3-one
17β-hydroxy-17α-(3-hydroxyprop-1(Z)-ènyl)-11β,19-[4-(3-pyridyl)-o-phénylène]-4,8-androstadiène-3-one
17β-hydroxy-17α-(3-hydroxyprop-1(Z)-ènyl)-11β,19-[4-(4-méthoxyphényl)-o-phénylène]-4,8-androstadiène-3-one
17β-hydroxy-17α-(3-hydroxyprop-(Z)-ènyl)-11β,19-[4-(4-méthylthiophényl)-o-phénylène]-4,8-androstadiène-3-one
11β,19-(4-acétyl-o-phénylène)-17β-hydroxy-17α-(3-hydroxyprop-1(Z)-ènyl)-4,8-androstadiène-3-one
11β,19-(4-acétyl-o-phénylène)-17β-hydroxy-17α-(prop-1-inyl)-4,8-androstadiène-3-one
17β-hydroxy-11β,19-[4-(4-méthylthiophényl)-o-phénylène)-17α-(prop-1-inyl)-4,8-androstadiène-3-one
17β-hydroxy-11β,19-[4-(3-furanyl)-o-phénylène]-17α-(prop-1-inyl)-4,8-androstadiène-3-one
17β-hydroxy-17α-(prop-1-inyl)-11β,19-[4-(3-pyridyl)-o-phénylène]-4,8-androstadiène-3-one
17β-hydroxy-17α-méthyl-11β,19-[4-(4-cyanophényl)-o-phénylène]-4,8-androstadiène-3-one
17α-cyanométhyl-17β-hydroxy-11β,19-[4-(4-cyanophényl)-o-phénylène]-4,8-androstadiène-3-one
17α-cyanométhyl-17β-hydroxy-11β,19-[4-(4-diméthylaminophényl)-o-phénylène]-4,8-androstadiène-3-one
17β-hydroxy-17α-(3-hydroxyprop-1(Z)-ènyl)-11β,19-(4-vinylphényl-o-phénylène]-4,8-androstadiène-3-one
17β-hydroxy-11β-,19-[4-(4-méthylthiophényl)-o-phénylène]-17α-(prop-1-inyl)-4,6-androstadiène-3-one
17β-hydroxy-17α-(3-hydroxyprop-1(Z)-ènyl)-11β,19-(4-méthylsulfinylphényl-o-phénylène)-4,8-androstadiène-3-one
17β-hydroxy-17α-(3-hydroxyprop-1(Z)-ènyl)-11β,19-(4-méthylthio-o-phénylène)-4,8-androstadiène-3-one
17β-hydroxy-17α-(3-hydroxyprop-1(Z)-ènyl)-11β,19-(4-méthylsulfinyl-o-phénylène)-4,8-androstadiène-3-one
11β,19-(4-diméthylamino-o-phénylène)-17β-hydroxy-17α-(3-hydroxyprop-1(Z)-ényl)-4,8-androstadiène-3-one
11β,19-[4-(4-diméthylaminophényl)-o-phénylène)-17β-hydroxy-17α-(3-hydroxyprop-1(Z)-ènyl)-4,8-androstadiène-3-one
11β,19-[4-(3-furyl)-o-phénylène)-17β-hydroxy-17α-(3-hydroxyprop-1(Z)-ènyl)-4,8-androstadiène-3-one
11β,19-[4-(4-cyanophényl)-o-phénylène)-spiro[androsta-4,8-diène-17β,2'(5'H)-furan]-3-one
11β,19-[4-(4-cyanophényl)-o-phénylène)-4',5'-dihydrospiro[androsta-4,8-diène-17β,2'(3'H)-furan]-3-one
11β,19-[4-(4-acétylphényl)-o-phénylène)-17β-hydroxy-17α-(3-hydroxyprop-1-inyl)-4,8-androstadiène-3-one
11β,19-[4-(4-acétylphényl)-o-phénylène)-17β-hydroxy-17α-(3-hydroxyprop-1(Z)-ènyl)-4,8-androstadiène-3-one
11β,19-[4-(4-acétylphényl)-o-phénylène]-spiro[androsta-4,8-diène-17β,2'(5'H)-furan]-3-one
11β,19-[4-(4-acétylphényl)-o-phénylène)-4',5'-dihydrospiro[androsta-4,8-diène-17β,2'(3'H)-furan]-3-one
11β,19-[4-(4-méthylthiophényl)-o-phénylène)-spiro[androsta-4,8-diène-17β,2'(5'H)-furan]-3-one
11β,19-[4-(4-méthylthiophényl)-o-phénylène)-4',5'-dihydrospiro[androsta-4,8-diène-17β,2'(3'H)-furan]-3-one
11β,19-(4-acétyl-o-phénylène)-spiro[androsta-4,8-diène-17β,2'(5'H)-furan]-3-one
11β,19-(4-acétyl-o-phénylène)-4',5'-dihydrospiro[androsta-4,8-diène-17β,2'(3'H)-furan]-3-one

**12.** Composé de formule générale III

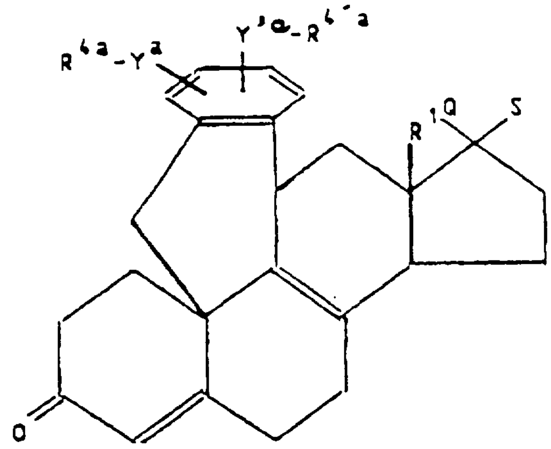

(III),

où

$R^{4a}$, $R^{4'a}$, $Y^a$ et $Y'^a$ ont les même significations que $R^4$, $R^{4'}$, Y et Y', des groupes hydroxy, mercapto, amino, oxo et/ou acétyl terminaux éventuellement présents étant protégés, ou $R^{4a}$-$Y^a$ et/ou $R^{4'}$-$Y'^a$ étant chacun un groupe méthoxy, hydroxy ou perfluoroalkylsulfonate, Q et S ensemble représentent un atome d'oxygène ou une combinaison de substituants énumérée sous $R^5/R^6$, qui subit sans dommages l'étape réactionnelle suivante de séparation de l'eau, ou dans laquelle les groupes hydroxyles libres sont protégés.

**13.** Procédé de préparation de composés de formule générale I

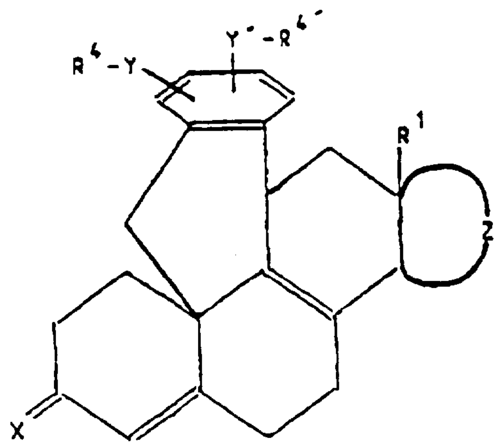

(I),

où

$R^1$, X, Z, $R^4$ et $R^{4'}$ ainsi que Y et Y' ont les significations déjà données dans la revendication 1, caractérisé en ce qu'on fait réagir un composé de formule générale II

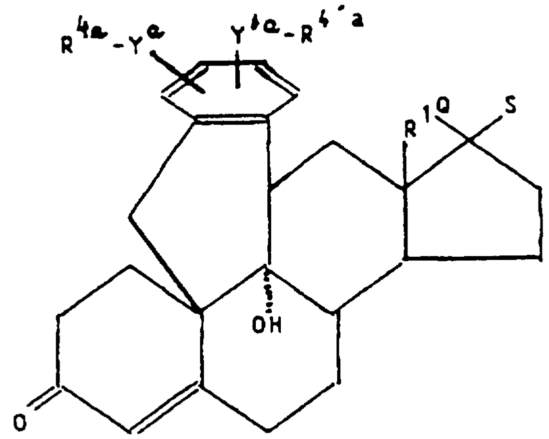

(II),

où

$R^{4a}$, $R^{4'a}$, $Y^a$ et $Y'^a$ ont les même significations que $R^4$, $R^{4'}$, Y et Y', des groupes hydroxy, mercapto, amino, oxo et/ou acétyl terminaux éventuellement présents étant protégés, ou
$R^{4a}$-$Y^a$ et/ou $R^{4'}$-$Y'^a$ étant chacun un groupe méthoxy, hydroxy ou perfluoroalkylsulfonate, Q et S ensemble représentent un atome d'oxygène ou une combinaison de substituants énumérée sous $R^5/R^6$, qui subit sans dommages l'étape réactionnelle suivante de séparation de l'eau, ou dans laquelle les groupes hydroxyles libres sont protégés,
en transformant par réaction avec un agent d'extraction d'eau en le composé 8-ène correspondant de formule générale III

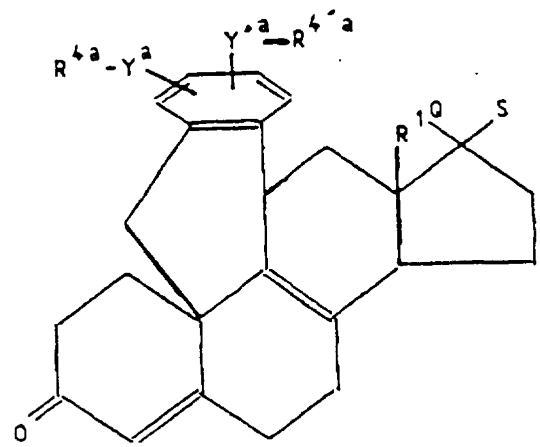

(III),

et ensuite, éventuellement,
lorsque Q et S ensemble représentent un atome d'oxygène, en protégeant sélectivement la fonction 3-céto sous la forme d'un diénoléther, d'un cétal ou d'un dithiocétal correspondant et en introduisant les substituants $R^5/R^6$ ou leurs précurseurs par addition nucléophile sur la fonction 17-céto de façon connue en soi (dégradation de la chaîne latérale), en libérant à nouveau le groupe 3-céto, puis éventuellement, lorsque $R^6$ doit représenter $-(CH_2)_m-CH_2-R^9$ ou $-CH=CH-(CH_2)_k-CH_2-R^9$, en hydrogénant catalytiquement le composés alcynyle correspondant, ainsi qu'éventuellement
lorsque $R^{4a}$-$Y^a$ et/ou $R^{4'a}$-$Y'^a$ représente un groupe perfluoroalkylsulfonate, lequel est éventuellement généré auparavant à partir d'un groupe méthoxy par l'intermédiaire d'un groupe hydroxyle, en transformant le composé de perfluoroalkylsulfonate soit directement, soit par échange du groupe éliminable perfluoroalkylsulfonato contre un groupe trialkylétain par l'intermédiaire du composé trialkylétain correspondant en un composé lequel présente, éventuellement à l'aide d'autres réactions, dans le cycle 19,11β-phényle la configuration de la substitution désirée, cette étape dernière de procédé citée, le couplage d'aryle, pouvant être effectuée dans n'importe quelle étape du procédé conforme à l'invention,
en libérant éventuellement le produit ainsi obtenu des groupes protecteurs si on le souhaite en alkylant, respectivement en acylant, le(s) groupes(s) hydroxy, mercapto et/ou amino, éventuellement contenu(s) dans le cycle 19,11β-phényle, si on le souhaite, en oxydant le(s) groupe(s) amino et/ou sulfur éventuellement contenu (s) dans le(s) substituant(s) aryle, si on le souhaite, en faisant réagir avec le chlorhydrate d'hydroxylamine pour obtenir le produit de formule générale I avec X ayant la signification d'un groupement hydroxylimino >N~OH, ainsi qu'éventuellement en préparant un sel d'addition d'acide pharmaceutiquement compatible.

**14.** Procédé selon la revendication 13, caractérisé en ce qu'on utilise le chlorure de thionyle comme agent d'extraction d'eau.

**15.** Préparations pharmaceutiques qui contiennent au moins un composé selon les revendications 1 à 11 ainsi qu'un support pharmaceutiquement compatible.

**16.** Utilisation de composés selon la revendication 1 à 11 pour la préparation de médicaments.

Tabelle 1

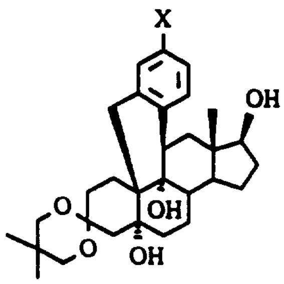

| X = | Fp. (kristallisiert aus) | $[\alpha]^{20}_D$ | (Solvens; Konzentation) |
|---|---|---|---|
| $OCH_3$ | 254-255 °C (EE) | 31 ° | ($CHCl_3$; 0,51) |
| OH | 242-244 °C (DIPE/$CH_2Cl_2$) | 32 ° | ($CHCl_3$; 0,51) |
| OTf | 176-178 °C (DIPE/$CH_2Cl_2$) | 29 ° | ($CHCl_3$; 0,51) |
| Cyanphenyl | 302-305 °C (EE) | 56 ° | ($CHCl_3$; 0,51) |

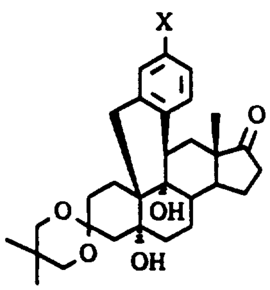

| X = | Fp. (kristallisiert aus) | $[\alpha]^{20}_D$ | (Solvens; Konzentation) |
|---|---|---|---|
| $OCH_3$ | 270-271 °C (MeOH/EE) | 46 ° | ($CHCl_3$; 0,5) |
| OTf | 205-208 °C (EE) | 40 ° | ($CHCl_3$; 0,51) |
| Vinyl | 273-275 °C (DIPE/$CH_2Cl_2$) | | |
| Et | 260-261 °C (EE) | 49 ° | ($CHCl_3$; 0,51) |
| Cyanphenyl | 296-299 °C (EE) | 72 ° | ($CHCl_3$; 0,515) |

EE = Ethylacetat / DIPE = Diisopropylether / MeOH = Methanol